(19) Europäisches Patentamt
European Patent Office
Office européen des brevets

(11) Publication number : **0 583 971 A1**

# (12) EUROPEAN PATENT APPLICATION

(21) Application number : **93306471.9**

(22) Date of filing : **17.08.93**

(51) Int. Cl.[5] : **C07D 211/74,** C08K 5/3435

(30) Priority : **17.08.92 JP 217902/92**

(43) Date of publication of application :
**23.02.94 Bulletin 94/08**

(84) Designated Contracting States :
**CH DE FR GB IT LI**

(71) Applicant : **SANKYO COMPANY LIMITED**
**5-1 Nihonbashi Honcho 3-chome**
**Chuo-ku, Tokyo 103 (JP)**

(72) Inventor : **Toda, Toshimasa, c/o Sankyo Company Limited**
**2-58, Hiromachi 1-chome, Shinagawa-ku**
**Tokyo 140 (JP)**

Inventor : **Naito, Satoru, c/o Sankyo Company Limited**
**2-58, Hiromachi 1-chome, Shinagawa-ku**
**Tokyo 140 (JP)**
Inventor : **Takahashi, Hiroaki, c/o Sankyo Company Limited**
**2-58, Hiromachi 1-chome, Shinagawa-ku**
**Tokyo 140 (JP)**
Inventor : **Ohsawa, Hisayu, c/o Sankyo Company Limited**
**2-58, Hiromachi 1-chome, Shinagawa-ku**
**Tokyo 140 (JP)**
Inventor : **Yamazaki, Takaaki, c/o Sankyo Company Limited**
**2-58, Hiromachi 1-chome, Shinagawa-ku**
**Tokyo 140 (JP)**

(74) Representative : **Gibson, Christian John Robert et al**
**MARKS & CLERK 57/60 Lincoln's Inn Fields**
**London WC2A 3LS (GB)**

(54) **Piperidine derivatives.**

(57) Compounds of formula (I) :

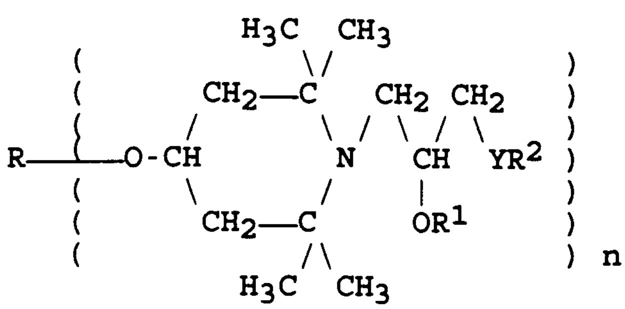

(I)

wherein :

R represents H or a mono- or poly- carboxylic acyl group ;

$R^1$ represents H or a carboxylic ester moiety ;

$R^2$ represents an alkyl group, an alkenyl group, an alkynyl group, an aryl group, or an aralkyl group, said groups being optionally substituted, or $R^2$ represents a carboxylic ester moiety ;

n = 1 - 4 ;

Y is O or S ;

and esters thereof,

are useful as heat and photo- stabilisers for polymeric materials.

EP 0 583 971 A1

The present invention relates to novel piperidine derivatives which impart excellent photostability to polymeric materials and to photostabilisers containing said piperidine derivatives as active ingredients.

2,2,6,6-Tetramethyl-4-piperidinol derivatives are commonly used as photostabilisers for synthetic resins and polymeric materials as well as for intermediates therefor.

In this context, a number of such 2,2,6,6-tetramethyl-4-piperidinol derivatives is known. For example, Japanese Patent Publication (Kokoku) No. Hei-3-1304 discloses a 2,3-dihydroxypropyl compound obtained by derivatising the 4-hydroxyl group of 2,2,6,6-tetramethyl-4-piperidinol with a monocarboxylic acid compound and derivatising the nitrogen at the 1-position with glycidol.

Japanese Patent Publication (Kokoku) No. Sho-62-42898 also discloses compounds wherein the 4-hydroxyl group of 2,2,6,6-tetramethyl-4-piperidinol is derivatised with a monocarboxylic acid compound, but wherein the nitrogen at the 1-position is derivatised by reaction with a bisglycidyl ether or a bisglycidyl ester.

However, compounds obtainable by

a) reacting the 4-hydroxyl group of 2,2,6,6-tetramethyl-4-piperidinol with a monocarboxylic acid and the 1-position nitrogen with a monoglycidyl ether or a monoglycidyl ester, or

b) reacting the 4-hydroxyl group of 2,2,6,6-tetramethyl-4-piperidinol with a polycarboxylic acid and the 1-position nitrogen with a glycidyl ether or a glycidyl ester,

have not previously been described, and such compounds form the basis of the present invention.

The compounds of the present invention unexpectedly exhibit greater heat and photo- resistance, and also demonstrate greater polymer affinity and less tendency toward creep and volatility.

Other 2,2,6,6-tetramethylpiperidine derivatives are disclosed in Japanese Patent No's 51-139841, 53-101380, 48-65180 and 57-136587.

Conventional 4-acyl derivatives of 2,2,6,6-tetramethyl-4-piperidinol described in the art are effective for preventing photodeterioration of synthetic resins or polymeric materials, particularly polyolefins. Unfortunately, they possess high volatility, are prone to surface migration and they suffer from poor compatibility with various polymers.

Accordingly, there is a need to provide new piperidine derivatives. There is also a need to provide photostabilisers which not only demonstrate excellent photostability and good polymer compatibility, but which also have reduced volatility and a reduced tendency for surface migration.

We have now surprisingly found that the addition products of 2,2,6,6-tetramethyl-4-hydroxypiperidine compounds with monoglycidyl derivatives overcome the above-described problems, in that they exhibit excellent photo- stability and good polymer compatibility, as well as having reduced volatility and a reduced tendency for surface migration.

Thus, in a first aspect, the present invention provides a compound of formula (I):

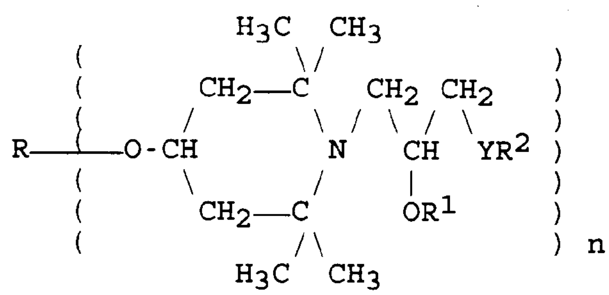

(I)

in which:

R represents a hydrogen atom, a monocarboxylic acyl group or a polycarboxylic acyl group, R having a valency of from 1 to 4;

$R^1$ represents a hydrogen atom or a carboxylic ester moiety,

$R^2$ represents:

- an alkyl group,
- an alkenyl group,
- an alkynyl group,
- an aryl group,
- an aralkyl group,
- a heterocyclic group,
- a heterocyclylalkyl group,

said aryl, aralkyl, heterocyclic and heterocyclylalkyl groups optionally having one or more substituents,

or $R^2$ represents a carboxylic ester moiety;
n is an integer between 1 and 4, inclusive, and is equal to the valency of R; and
Y represents an oxygen atom or a sulphur atom,
**PROVIDED THAT,** when n is 2, 3 or 4, then each $R^1$ and each $R^2$ may be the same or different,
and acid addition salts thereof.

The present invention also provides photostabilisers for polymeric materials having at least one compound of formula (I) as an active ingredient.

The compounds of the present invention have excellent heat and photo- stabilising effects. In addition they exibit minimal volatility in situ, and show reduced surface migration, as well as being highly polymer compatible.

In the compounds of formula (I), we prefer that $R^2$ represents:
an alkyl group having from 1 to 18 carbon atoms,
an alkenyl group having from 2 to 6 carbon atoms,
an alkynyl group having from 2 to 6 carbon atoms,
an aryl group having from 6 to 10 carbon atoms, or
an aralkyl group having from 7 to 12 carbon atoms,
said groups being optionally substituted.

Where $R^2$ represents an alkenyl or an alkynyl group, then it is preferred that these groups are selected from:
an alkenyl group having from 3 to 6 carbon atoms, and
an alkynyl group having from 3 to 6 carbon atoms.

It will be appreciated that any aryl or heterocyclic group in the compounds of formula (I) can be substituted by any appropriate substituents, and that such substituents will be apparent to those skilled in the art. However, we prefer that the substituents are selected from substituents A, wherein substituents A are selected from:
alkyl groups having from 1 to 4 carbon atoms,
cycloalkyl groups having from 3 to 10 carbon atoms,
haloalkyl groups having from 1 to 4 carbon atoms,
halogen atoms,
aralkyl groups wherein the aryl part has from 6 to 10 carbon atoms and the alkyl part has from 1 to 4 carbon atoms,
hydroxy groups,
alkoxy groups having from 1 to 4 carbon atoms,
nitro groups,
cyano groups,
carbamoyl groups,
carboxy groups, and
amino groups which are optionally substituted by at least one alkyl group having from 1 to 4 carbon atoms.

More preferably, substituents A are selected from:
alkyl groups having from 1 to 4 carbon atoms,
cycloalkyl groups having from 3 to 6 carbon atoms,
haloalkyl groups having 1 or 2 carbon atoms,
halogen atoms,
aralkyl groups wherein the aryl part has from 6 to 10 carbon atoms and the alkyl part has 1 or 2 carbon atoms and
hydroxyl groups.

In the compounds of formula (I), when R represents a monocarboxylic acyl group, then suitable acyl groups may be selected from alkanoyl groups, preferably having from 1 to 30, more preferably from 1 to 18, carbon atoms. These may be straight or branched chain groups, and examples include: the formyl, acetyl, propionyl, butyryl, isobutyryl, valeryl, isovaleryl, pivaloyl, 2-methylbutyryl, hexanoyl, isohexanoyl, 3-methylvaleryl, 4,4-dimethylbutyryl, 2-ethylbutyryl, heptanoyl, 5-methylhexanoyl, 4-methylhexanoyl, 3-methylhexanoyl, 2-methylhexanoyl, 4,4-dimethylvaleryl, 3,3-dimethylvaleryl, 2,2-dimethylvaleryl, 2,3-dimethylvaleryl, 2,4-dimethylvaleryl, 3,4-dimethylvaleryl, 3-ethylvaleryl, octanoyl, 2-methylheptanoyl, 3-methylheptanoyl, 4-methylheptanoyl, 5-methylheptanoyl, 6-methylheptanoyl, 2-propylvaleryl, 5,5-dimethylhexanoyl, nonanoyl, 2-methyloctanoyl, 3-methyloctanoyl, 4-methyloctanoyl, 5-methyloctanoyl, 6-methyloctanoyl, 7-methyloctanoyl, 2-propylhexanoyl, 3-ethylheptanoyl, 6,6-dimethylheptanoyl, decanoyl, 4-methylnonanoyl, 5-methylnonanoyl, 6-methylnonanoyl, 7-methylnonanoyl, 2-propylheptanoyl, 3-ethyloctanoyl, 7,7-dimethyloctanoyl, undecanoyl, 2-methyldecanoyl, 4-methyldecanoyl, 9-methyldecanoyl, 4-ethylnonanoyl, 4,8-dimethylnonanoyl, 8,8-dimethylnonanoyl, lauroyl, 4,8-dimethyldecanoyl, tridecanoyl, myristoyl, pentadecanoyl, palmitoyl, 3,7,11-trimethyltridecanoyl, heptadecanoyl, 4,8,12-trimethylmyristoyl, 1-methylpalmitoyl, 14-methylpalmitoyl, 13,13-dimethylpentadecanoyl, stearoyl, 13,13-dimethylpentadecanoyl, stearoyl, 15-methylheptadecanoyl, nonadecanoyl, 1-methylstearoyl, icosanoyl, henicosanoyl, 3,7,11,15-tetramethylheptadecanoyl, docosanoyl and tricosanoyl groups.

More preferred alkanoyl groups include: the formyl, acetyl, propionyl, butyryl, valeryl, isovaleryl, pivaloyl, hexanoyl, heptanoyl, octanoyl, decanoyl, undecanoyl, lauroyl, myristoyl, palmitoyl and stearoyl groups.

Other suitable acyl groups represented by R include: alkenoyl groups, preferably having from 3 to 6 carbon atoms, for example the acryloyl, methacryloyl, crotonoyl, isocrotonoyl and (E)-2-methyl-2-butenoyl groups; cycloalkanoyl groups having 6 or 7 carbon atoms, such as the cyclopentanecarbonyl and cyclohexanecarbonyl groups; arylcarbonyl groups having from 7 to 11 carbon atoms, the aryl portion of which may be substituted as described above, or more preferably, with from 1 to 3 alkyl groups having from 1 to 4 carbon atoms which may be the same or different from one another, such as the benzoyl, toluoyl, xyloyl, ethylbenzoyl, propylbenzoyl, butylbenzoyl, naphthoyl, benziloyl, salicyloyl, anisoyl, vanilloyl, veratroyl, protocatechuoyl, galloyl and picryl groups; and 5- or 6-membered aromatic heterocyclic acyl groups which may contain 1 or 2 hetero atoms, which may be the same or different from each other, and which are selected from nitrogen, oxygen and sulphur, such as the picolinoyl, nicotinoyl, isonicotinoyl, pyrazinecarbonyl, thenoyl and furoyl groups.

When R represents a dicarboxylic acyl group, then suitable acyl groups may generally be selected from the diacyl groups corresponding to those listed above or, more preferably, are selected from: an alkanedioyl group having from 3 to 18 carbon atoms, such as the malonyl, 1,1-diethylmalonyl, succinyl, glutaryl, adipoyl, pimeloyl, suberoyl, azelaoyl, sebacoyl, undecanedioyl, dodecanedioyl, tridecanedioyl, tetradecanedioyl, hexadecanedioyl, octadecanedioyl, diglycoloyl and thiodiglycoloyl groups; a cycloalkanedioyl group having from 8 to 12 carbon atoms or cycloalkenedioyl group having from 8 to 12 carbon atoms, such as the cyclohexanedicarbonyl, cyclohexenedicarbonyl, norbornenedicarbonyl and camphoroyl groups; an aryldiacyl group having from 8 to 12 carbon atoms, such as the phthaloyl, isophthaloyl, terephthaloyl and naphthalenedicarbonyl groups; and a 5- or 6-membered aromatic heterocyclic diacyl group which may contain 1 or 2 hetero-atoms, each of which may be the same or different and which are selected from nitrogen, oxygen and sulphur, such as the pyridinedicarbonyl and thiophenedicarbonyl groups.

When R represents a tricarboxylic acyl group, then suitable acyl groups may generally be selected from the triacyl groups corresponding to those listed above or, more preferably, are selected from: an alkanetriacyl group containing 6 carbon atoms, such as the 1,2,3-propanetricarbonyl group; and an aryltriacyl group having from 9 to 13 carbon atoms, such as the benzenetricarbonyl and naphthalenetricarbonyl groups.

When R represents a tetracarboxylic acyl group, then suitable acyl groups may generally be selected from the tetraacyl groups corresponding to those listed above or, more preferably, are selected from: an alkanetetraacyl group containing 8 carbon atoms, such as the 1,2,3,4-butanetetracarbonyl group; or an aryltetraacyl group containing 10 carbon atoms, such as the benzenetetracarbonyl group.

In the compounds of formula (I), preferred meanings for R include: an alkanoyl group having from 2 to 10 carbon atoms; an alkanedioyl group having from 3 to 14 carbon atoms; and a benzoyl group optionally having from 1 to 3 alkyl substituents having from 1 to 4 carbon atoms and which may be the same or different from one another.

More preferably, R represents: an alkanoyl group having from 2 to 4 carbon atoms; an alkanedioyl group having from 6 to 12 carbon atoms; or a benzoyl group optionally having 1 or 2 methyl or ethyl substituents which may be the same or different from each other.

We particularly prefer R to represent: an acetyl group; an alkanedioyl group having from 8 to 12 carbon atoms; or a benzoyl group optionally having 1 or 2 methyl or ethyl substituents which may be the same or different from each other.

Most preferably R represents an unsubstituted alkanedioyl group having from 8 to 10 carbon atoms.

It will be appreciated that, when $R^1$ and/or $R^2$ represent an ester moiety, then any appropriate ester moiety may be chosen. Preferred ester moieties are indicated below, but the only limitation is that the they should not reduce, at least not to a disadvantageous degree, the useful properties of the compounds of the present invention.

When $R^1$ and/or $R^2$ in the compounds of formula (I) represents an ester moiety, then suitable ester moieties are preferably selected from:

alkanoyl groups having from 1 to 18 carbon atoms,

haloalkanoyl groups having from 2 to 4 carbon atoms,

arylcarbonyl groups having from 7 to 11 carbon atoms, and

arylalkanoyl groups having from 8 to 13 carbon atoms, the aryl parts of said arylcarbonyl and arylalkanoyl groups being optionally substituted by at least one substituent individually selected from substituents A.

Where any aryl or heterocyclyl group is substituted by a substituent A, then the preferred number of substituents is from 1 to 5, with 1, 2 or 3 being more preferred, although it will be appreciated that the number of substituents is not necessarily restricted to a maximum of 5, and may be as many as is desired, subject only to available positions on the group to be substituted, and steric considerations, where appropriate.

When $R^1$ and/or $R^2$ of the compounds of formula (I) represent an alkanoyl ester group, then this preferably has from 1 to 18 carbon atoms, and suitable groups may be selected from those groups defined for R, above, when unsubstituted. More preferably, suitable groups are selected from: the formyl, acetyl, propionyl, butyryl, isobutyryl, pivaloyl, valeryl, isovaleryl, hexanoyl, heptanoyl, octanoyl, nonanoyl, decanoyl, undecanoyl, lauroyl, myristoyl, palmitoyl and stearoyl groups. Preferred groups are alkanoyl groups having from 2 to 10 carbon atoms, especially alkanoyl groups having from 2 to 6 carbon atoms. Particularly preferred are alkanoyl groups having from 2 to 4 carbon atoms, and most preferred is the acetyl group.

When $R^1$ and/or $R^2$ of the compounds of formula (I) represent an arylcarbonyl moiety, then the aryl part of this preferably has from 6 to 10 ring carbon atoms, and suitable groups include the benzoyl and naphthoyl groups, preferably a benzoyl group.

When $R^1$ and/or $R^2$ of the compounds of formula (I) represent an optionally substituted arylalkanoyl moiety, then this preferably has from 8 to 13 carbon atoms, and suitable examples include: an arylalkanoyl group having from 2 to 3 carbon atoms substituted with an aryl group having from 6 to 10 carbon atoms, such as the phenylacetyl, 3-phenylpropionyl, 1-naphthylacetyl, 2-naphthylacetyl and 3-(1-naphthyl)propionyl groups. The arylalkanoyl moiety is preferably an arylalkanoyl group having from 8 to 12 carbon atoms, more preferably an arylalkanoyl group having 8 or 9 carbon atoms, and still more preferably an arylalkanoyl group having 9 carbon atoms, particularly the 3-phenylpropionyl group.

When $R^1$ and/or $R^2$ of the compounds of formula (I) represent a haloalkanoyl group, this preferably has from 2 to 4 carbon atoms, and suitable examples include: the trifluoroacetyl, tribromoacetyl, trichloroacetyl, monobromoacetyl, monochloroacetyl, dichloroacetyl, 2,3-dichloropropionyl and 2-bromoisobutyryl groups. It is preferred that the haloalkanoyl group has from 2 to 4 carbon atoms, and is particularly preferably a fluoroacetyl group.

When $R^1$ and/or $R^2$ of the compounds of formula (I) represent an alkyl group, then this may be straight or branched chain, and preferably has from 1 to 30, more preferably from 1 to 18, carbon atoms, and suitable examples include: the methylethyl, propyl, isopropyl, butyl, isobutyl, pentyl, neo-pentyl, 2-ethylhexyl, hexyl, heptyl, octyl, nonyl, decyl, undecyl, dodecyl, tridecyl, tetradecyl, pentadecyl, hexadecyl, heptadecyl and octadecyl groups. Preferably, the alkyl group has from 1 to 10 carbon atoms, more preferably 1 to 4 carbon atoms. The methyl group is particularly preferred.

When $R^1$ and/or $R^2$ of the compounds of formula (I) represent an alkenyl group, this preferably has from 2 to 6 carbon atoms, and suitable examples include: the vinyl, allyl, 1-butenyl, 2-butenyl, 2-methyl-2-butenyl, 1-pentenyl, 1-hexenyl and 2-hexenyl groups. Straight chain alkenyl groups having from 3 to 6 carbon atoms are preferred, and the allyl group is most preferred.

When $R^1$ and/or $R^2$ of the compounds of formula (I) represent an alkynyl group, this preferably has from 3 to 6 carbon atoms, and suitable examples include: the 1-butynyl, 2-butynyl, 2-methyl-2-butynyl, 1-pentynyl, 1-hexynyl and 2-hexynyl groups.

When $R^1$ and/or $R^2$ of the compounds of formula (I) represent an optionally substituted aryl moiety, suitable examples include: aryl groups substituted with a halogen atom or atoms, such as the 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 3,5-difluorophenyl, 2,5-difluorophenyl, 2,6-difluorophenyl, 2,4-difluorophenyl, 3,5-dibromophenyl, 2,5-dibromophenyl, 2,6-dichlorophenyl, 2,4-dichlorophenyl, 2,3,6-trifluorophenyl, 2,3,4-trifluorophenyl, 3,4,5-trifluorophenyl, 2,5,6-trifluorophenyl, 2,4,6-trifluorophenyl, 2,3,6-tribromophenyl, 2,3,4-tribromophenyl, 3,4,5-tribromophenyl, 2,5,6-trichlorophenyl, 2,4,6-trichlorophenyl, 1-fluoro-2-naphthyl, 2-fluoro-1-naphthyl, 3-fluoro-1-naphthyl, 1-chloro-2-naphthyl, 2-chloro-1-naphthyl, 3-bromo-1-naphthyl, 3,8-difluoro-1-naphthyl, 2,3-difluoro-1-naphthyl, 4,8-difluoro-1-naphthyl, 5,6-difluoro-1-naphthyl, 3,8-dichloro-1-naphthyl, 2,3-dichloro-1-naphthyl, 4,8-dibromo-1-naphthyl, 5,6-dibromo-1-naphthyl, 2,3,6-trifluoro-1-naphthyl, 2,3,4-trifluoro-1-naphthyl, 3,4,5-trifluoro-1-naphthyl, 4,5,6-trifluoro-1-naphthyl and 2,4,8-trifluoro-1-naphthyl groups; aryl groups substituted with a haloalkyl group or groups, such as the 2-trifluoromethylphenyl, 3-trifluoromethylphenyl, 4-trifluoromethylphenyl, 2-trichloromethylphenyl, 3-dichloromethylphenyl, 4-trichloromethylphenyl, 2-tribromomethylphenyl, 3-dibromomethylphenyl, 4-dibromomethylphenyl, 3,5-bistrifluoromethylphenyl, 2,5-bistrifluoromethylphenyl, 2,6-bistrifluoromethylphenyl, 2,4-bistrifluoromethylphenyl, 3,5-bistribromomethylphenyl, 2,5-bisdibromomethylphenyl, 2,6-bisdichloromethylphenyl, 2,4-bisdichloromethylphenyl, 2,3,6-tristrifluoromethylphenyl, 2,3,4-tristrifluoromethylphenyl, 3,4,5-tristrifluoromethylphenyl, 2,5,6-tristrifluoromethylphenyl, 2,4,6-tristrifluoromethylphenyl, 2,3,6-tristribromomethylphenyl, 2,3,4-trisdibromomethylphenyl, 3,4,5-tristribromomethylphenyl, 2,5,6-trisdichloromethylphenyl, 2,4,6-trisdichloromethylphenyl, 1-trifluoromethyl-2-naphthyl, 2-trifluoromethyl-1-naphthyl, 3-trifluoromethyl-1-naphthyl, 1-trichloromethyl-2-naphthyl, 2-dichloromethyl-1-naphthyl, 3-tribromomethyl-1-naphthyl, 3,8-bistrifluoromethyl-1-naphthyl, 2,3-bistrifluoromethyl-1-naphthyl, 4,8-bistrifluoromethyl-1-naphthyl, 5,6-bistrifluoromethyl-1-naphthyl, 3,8-bistrichloromethyl-1-naphthyl, 2,3-bisdichloromethyl-1-naphthyl, 4,8-bisdibromomethyl-1-naphthyl, 5,6-bistribromomethyl-1-naphthyl, 2,3,6-tristrifluoromethyl-1-naphthyl, 2,3,4-tristrifluoromethyl-1-naphthyl, 3,4,5-tristrifluoromethyl-1-naphthyl, 4,5,6-tristrifluoromethyl-1-naphthyl and 2,4,8-tristrifluoromethyl-1-naphthyl groups; aryl groups substituted with an alkyl group or groups, such as the 2-methylphenyl, 3-methylphenyl, 4-methylphenyl, 2-ethylphenyl, 3-propylphenyl, 4-ethylphenyl, 2-butylphenyl, 3-pentylphenyl, 4-pentylphenyl, 3,5-dimethylphenyl, 2,5-dimethylphenyl, 2,6-dimethylphenyl, 2,4-dimethylphenyl, 3,5-dibutylphenyl, 2,5-dipentylphenyl, 2,6-dipropylphenyl, 2,4-dipropylphenyl, 2,3,6-trimethylphenyl, 2,3,4-trimethylphenyl, 3,4,5-trimethylphenyl, 2-t-butyl-6-methylphenyl, 2-t-butylphenyl, 4-t-butylphenyl, 2,5,6-trimethylphenyl, 2,4,6-trimethylphenyl, 2,3,6-tributylphenyl, 2,3,4-tripentylphenyl, 3,4,5-tributylphenyl, 2,5,6-tripropylphenyl, 2,4,6-tripropylphenyl, 1-methyl-2-naphthyl, 2-methyl-1-naphthyl, 3-methyl-1-naphthyl, 1-ethyl-2-naphthyl, 2-propyl-1-naphthyl, 3-butyl-1-naphthyl, 3,8-dimethyl-1-naphthyl, 2,3-dimethyl-1-naphthyl, 4,8-dimethyl-1-naphthyl, 5,6-dimethyl-1-naphthyl, 3,8-diethyl-1-naphthyl, 2,3-dipropyl-1-naphthyl, 4,8-dipentyl-1-naphthyl, 5,6-dibutyl-1-naphthyl, 2,3,6-trimethyl-1-naphthyl, 2,3,4-trimethyl-1-naphthyl, 3,4,5-trimethyl-1-naphthyl, 4,5,6-trimethyl-1-naphthyl and 2,4,8-trimethyl-1-naphthyl groups; aryl groups substituted with an alkoxy group or groups, such as the 2-methoxyphenyl, 3-methoxyphenyl, 4-methoxyphenyl, 2-ethoxyphenyl, 3-propoxyphenyl, 4-ethoxyphenyl, 2-butoxyphenyl, 3-pentyloxyphenyl, 4-pentyloxyphenyl, 3,5-dimethoxyphenyl, 2,5-dimethoxyphenyl, 2,6-dimethoxyphenyl, 2,4-dimethoxyphenyl, 3,5-dibutoxyphenyl, 2,5-dipentyloxyphenyl, 2,6-dipropoxyphenyl, 2,4-dipropoxyphenyl, 2,3,6-trimethoxyphenyl, 2,3,4-trimethoxyphenyl, 3,4,5-trimethoxyphenyl, 2,5,6-trimethoxyphenyl, 2,4,6-trimethoxyphenyl, 2,3,6-tributoxyphenyl, 2,3,4-tripentyloxyphenyl, 3,4,5-tributoxyphenyl, 2,5,6-tripropoxyphenyl, 2,4,6-tripropoxyphenyl, 1-methoxy-2-naphthyl, 2-methoxy-1-naphthyl, 3-methoxy-1-naphthyl, 1-ethoxy-2-naphthyl, 2-propoxy-1-naphthyl, 3-butoxy-1-naphthyl, 3,8-dimethoxy-1-naphthyl, 2,3-dimethoxy-1-naphthyl, 4,8-dimethoxy-1-naphthyl, 5,6-dimethoxy-1-naphthyl, 3,8-diethoxy-1-naphthyl, 2,3-dipropoxy-1-naphthyl, 4,8-dipentyloxy-1-naphthyl, 5,6-dibutoxy-1-naphthyl, 2,3,6-trimethoxy-1-naphthyl, 2,3,4-trimethoxy-1-naphthyl, 3,4,5-trimethoxy-1-naphthyl, 4,5,6-trimethoxy-1-naphthyl and 2,4,8-trimethoxy-1-naphthyl groups; aryl groups substituted with an amino group or groups, such as the 2-aminophenyl, 3-aminophenyl, 4-aminophenyl, 3,5-diaminophenyl, 2,5-diaminophenyl, 2,6-diaminophenyl, 2,4-diaminophenyl, 2,3,6-triaminophenyl, 2,3,4-triaminophenyl, 3,4,5-triaminophenyl, 2,5,6-triaminophenyl, 2,4,6-triaminophenyl, 1-amino-2-naphthyl, 2-amino-1-naphthyl, 3-amino-1-naphthyl, 3,8-diamino-1-naphthyl, 2,3-diamino-1-naphthyl, 4,8-diamino-1-naphthyl, 5,6-diamino-1-naphthyl, 2,3,6-triamino-1-naphthyl, 2,3,4-triamino-1-naphthyl, 3,4,5-triamino-1-naphthyl, 4,5,6-triamino-1-naphthyl and 2,4,8-triamino-1-naphthyl groups; aryl groups substituted with a hydroxy group or groups, such as the 2-hydroxyphenyl, 3-hydroxyphenyl, 4-hydroxyphenyl, 3,5-dihydroxyphenyl, 2,5-dihydroxyphenyl, 2,6-dihydroxyphenyl, 2,4-dihydroxyphenyl, 2,3,6-trihydroxyphenyl, 2,3,4-trihydroxyphenyl, 3,4,5-trihydroxyphenyl, 2,5,6-trihydroxyphenyl, 2,4,6-trihydroxyphenyl, 1-hydroxy-2-naphthyl, 2-hydroxy-1-naphthyl, 3-hydroxy-1-naphthyl, 3,8-dihydroxy-1-naphthyl, 2,3-dihydroxy-1-naphthyl, 4,8-dihydroxy-1-naphthyl, 5,6-dihydroxy-1-naphthyl, 2,3,6-trihydroxy-1-naphthyl, 2,3,4-trihydroxy-1-naphthyl, 3,4,5-trihydroxy-1-naphthyl, 4,5,6-trihydroxy-1-naphthyl and 2,4,8-trihydroxy-1-naphthyl groups; aryl groups substituted with a cyano group or groups, such as the 2-cyanophenyl, 3-cyanophenyl, 4-cyanophenyl, 3,5-dicyanophenyl, 2,5-dicyanophenyl, 2,6-dicyanophenyl, 2,4-dicyanophenyl, 2,3,6-tricyanophenyl, 2,3,4-tricyanophenyl, 3,4,5-tricyanophenyl, 2,5,6-tricyanophenyl, 2,4,6-tricyanophenyl, 1-cyano-2-naphthyl, 2-cyano-1-naphthyl, 3-cyano-1-naphthyl, 3,8-dicyano-1-naphthyl, 2,3-dicyano-1-naphthyl, 4,8-dicyano-1-naphthyl, 5,6-dicyano-1-naphthyl, 2,3,6-tricyano-1-naphthyl, 2,3,4-tricyano-1-naphthyl, 3,4,5-tricyano-1-naphthyl, 4,5,6-tricyano-1-naphthyl and 2,4,8-tricyano-1-naphthyl groups; aryl groups substituted with a carboxyl group or groups, such as the 2-carboxyphenyl, 3-carboxyphenyl, 4-carboxyphenyl, 3,5-dicarboxyphenyl, 2,5-dicarboxyphenyl, 2,6-dicarboxyphenyl and 2,4-dicarboxyphenyl groups; aryl groups substituted with aralkyl groups, such as the 4-benzylphenyl and 4-cumylphenyl groups; and aryl groups substituted with a carbamoyl group or groups, such as the 2-carbamoylphenyl, 3-carbamoylphenyl, 4-carbamoylphenyl, 3,5-dicarbamoylphenyl, 2,5-dicarbamoylphenyl, 2,6-dicarbamoylphenyl and 2,4-dicarbamoylphenyl groups. The aryl group may also have any combination of two or more different substituents selected from the above list, for example the 3,5-di-t-butyl-4-hydroxyphenyl and 3-t-butyl-5-methyl-4-hydroxyphenyl groups.

More particularly, when $R^1$ and/or $R^2$ of the compounds of formula (I) represent an optionally substituted aryl moiety, then it is preferred that said groups are selected from: the phenyl, 2-tolyl, 2,6-xylyl, 4-t-butylphenyl, 2-t-butylphenyl, 2-t-butyl-6-methylphenyl, 3,5-di-t-butyl-4-hydroxyphenyl, 4-cumylphenyl and benzyl groups. The phenyl and 4-t-butylphenyl groups are most preferred.

In general, the unsubstituted aryl groups are preferred. More preferred are those unsubstituted aryl groups which have from 6 to 10 carbon atoms, more preferably 6 or 10 carbon atoms, especially the phenyl and naphthyl groups, most preferably the phenyl group.

When $R^1$ and/or $R^2$ of the compounds of formula (I) represent an optionally substituted aralkyl moiety, then suitable examples include: the benzyl, α-naphthylmethyl, β-naphthylmethyl, 2-methylbenzyl, 3-methylbenzyl, 4-methylbenzyl, 2,4,6-trimethylbenzyl, 3,4,5-trimethylbenzyl, 2-methoxybenzyl, 3-methoxybenzyl, 4-methoxybenzyl, 3,4-dimethoxybenzyl, 2-nitrobenzyl, 4-nitrobenzyl, 2-chlorobenzyl, 3-chlorobenzyl, 4-chlorobenzyl, 4-bromobenzyl, 4-cyanobenzyl, α or β-naphthylmethyl, benzhydryl (i.e. diphenylmethyl), trityl (i.e. triphenylmethyl), phenethyl, 1-phenylethyl, 1- (α or β- naphthyl)ethyl, 2-(α or βnaphthyl)ethyl, 1-phenylpropyl, 2-phenylpropyl, 3-phenylpropyl, 1-(α or β- naphthyl)propyl, 2-(α or β- naphthyl)propyl, 3-(α or β- naphthyl)propyl, 1-phenylbutyl, 2-phenylbutyl, 3-phenylbutyl, 4-phenylbutyl, 1-(α or β-naphthyl)butyl, 2-(α or β- naphthyl)butyl, 3-(α or β- naphthyl)butyl, 4-(α or β- naphthyl)butyl, 1-phenylpentyl, 2-phenylpentyl, 3-phenylpentyl, 4-phenylpentyl, 5-phenylpentyl, 1-(α or β-naphthyl)pentyl, 2-(α or β- naphthyl)pentyl, 3-(α or β- naphthyl)pentyl, 4-(α or β- naphthyl)pentyl, 5-(α or β- naphthyl)pentyl, 1-phenylhexyl, 2-phenylhexyl, 3-phenylhexyl, 4-phenylhexyl, 5-phenylhexyl, 6-phenylhexyl, 1-(α or β-naphthyl)hexyl, 2-(α or β- naphthyl)hexyl, 3-(α or β- naphthyl)hexyl, 4-(α or β- naphthyl)hexyl, 5-(α or β- naphthyl)hexyl and 6-(α or β-naphthyl)hexyl groups. It is preferred that such groups have from 7 to 12 carbon atoms, and preferred examples include: an alkyl group having from 1 to 3 carbon atoms substituted with an aryl group having from 6 to 10 carbon atoms, such as the benzyl, 2-phenylethyl, 3-phenylpropyl, naphthyl-1-methyl and naphthyl-2-methyl groups. Preferred are $C_6$-aryl($C_1$ - $C_3$)alkyl groups, particularly $C_6$ aryl($C_1$ - $C_2$)alkyl groups. The most preferred group is benzyl.

The heterocyclic groups and the heterocyclic part of the heterocyclylalkyl groups defined herein have from 3 to 14 ring atoms of which from 1 to 4 are hetero-atoms selected from nitrogen, oxygen and sulphur hetero-atoms and of which the remaining ring atoms are carbon atoms, said heterocyclic groups being unsubstituted or being substituted by at least one substituent selected from substituents A. In the case of those groups having 4 hetero-atoms in a ring, we prefer that 3 or 4 of them should be nitrogen atoms and 1 or 0 should be an oxygen or sulphur atom. In the case of those groups having 3 hetero-atoms in a ring, we prefer that 1, 2 or 3 should be nitrogen atoms and, correspondingly, 2, 1 or 0 should be oxygen and/or sulphur atoms. In the case of those groups having 1 or 2 hetero-atoms in a ring, the hetero-atoms may be freely chosen from nitrogen, oxygen and sulphur atoms. Preferably, however, the group contains at least one nitrogen atom.

More specifically, $R^2$ may represent a heterocyclic group and, where $R^2$ represents an aromatic heterocyclic group, then this must necessarily have at least 5 ring atoms and thus have from 5 to 10 ring atoms, more preferably from 5 to 7 ring atoms. The groups also preferably contain from 1 to 3 hetero-atoms selected from nitrogen, oxygen and sulphur atoms. Suitable examples include: the thienyl, thianthrenyl, furyl, pyranyl, isobenzofuranyl, chromenyl, xanthenyl, phenoxathiinyl, 2H-pyrrolyl, pyrrolyl, imidazolyl, pyrazolyl, isothiazolyl, isoxazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinoxalinyl, quinazolinyl, cinnolinyl, pteridinyl, 4aH-carbazolyl, carbazolyl, β-carbolinyl, phenanthridinyl, acridinyl, perimidinyl, phenanthrolinyl, phenazinyl, phenothiazinyl, furazanyl, phenoxazinyl, isochromanyl, chromanyl, pyrrolidinyl, pyrrolinyl, imidazolidinyl, imidazolinyl, pyrazolidinyl, pyrazolinyl, piperidyl, piperazinyl, indolinyl, isoindolinyl, quinuclidinyl, morpholinyl, azepinyl, oxazolyl, thiazolyl, 1,2,3-oxadiazolyl, triazolyl, tetrazolyl and thiadiazolyl groups. Of these, we prefer those aromatic heterocyclic groups containing 5 or 6 ring atoms of which from 1 to 3 are sulphur, oxygen or/and nitrogen atoms or containing 4 ring nitrogen atoms. The most preferred aromatic heterocyclic groups are those containing 5 or 6 ring atoms of which at least one is a nitrogen atom, and which may optionally contain an oxygen or sulphur atom or atoms, or containing 4 ring nitrogen atoms. Examples of preferred groups include: the pyrrolyl, azepinyl, pyrazolyl, imidazolyl, oxazolyl, isoxazolyl, thiazolyl, isothiazolyl, 1,2,3-oxadiazolyl, triazolyl, tetrazolyl, thiadiazolyl, pyridyl, pyridazinyl, pyrimidinyl and pyrazinyl groups. More preferred examples include: the imidazolyl, oxazolyl, isoxazolyl and thiazolyl groups.

Where $R^2$ represents a heterocyclylalkyl group, then the heterocyclic portion may suitably be selected from those heterocyclic groups defined above, while the alkyl portion preferably has from 1 to 6 carbon atoms, more preferably from 1 to 4 carbon atoms, and especially 1 or 2 carbon atoms.

Suitable examples for the alkyl group having from 1 to 4 carbon atoms in substituents A include: the methyl, ethyl, propyl, isopropyl, butyl, isobutyl, sec-butyl and t-butyl groups. Preferred are the methyl, ethyl, isopropyl and t-butyl groups, and more preferred are the methyl and t-butyl groups. Most preferred is the t-butyl group.

Suitable examples for the cycloalkyl group having from 3 to 6 carbon atoms in substituents A include: the cyclopropyl, cyclobutyl, cyclopentyl and cyclohexyl groups. Preferred are the cyclopropyl and cyclobutyl groups, with the cyclopropyl group being most preferred.

Suitable examples for the haloalkyl group having from 1 to 4 carbon atoms in substituents A include: the fluoromethyl, chloromethyl, bromomethyl, trifluoromethyl, trichloromethyl, tribromomethyl, trifluoromethyl, 2,2,2-trifluoroethyl, 2,2,2-trichloroethyl, 2,2,2-tribromoethyl, 3,3,3-trifluoropropyl, 3,3,3-trichloropropyl, 3,3,3-tribromopropyl, 4,4,4-trifluorobutyl, and 4,4,4-trichlorobutyl, 4,4,4-tribromobutyl groups. Preferred are fluoroalkyl and chloroalkyl groups having 1 or 2 carbon atoms, with the trifluoromethyl group being most preferred.

Suitable examples for the halogen atom in substituents A include: fluorine, chlorine, bromine and iodine. Preferred are fluorine, chlorine and bromine. Fluorine and chlorine are most preferred.

Suitable examples for the aralkyl group wherein the aryl part has from 6 to 10 carbon atoms and the alkyl part has from 1 to 4 carbon atoms in substituents A may be selected, as appropriate, from those listed above for $R^2$, but more preferably include: an aryl group having from 6 to 10 carbon atoms linked to an alkyl group having from 1 to 3 carbon atoms, such as the benzyl, 1-phenylethyl, 2-phenylethyl, 3-phenylpropyl, 2-phenylpropyl, α,α-dimethylphenylmethyl (cumyl), 1-naphthylmethyl, 2-(1-naphthyl)ethyl and 1-(1-naphthyl)ethyl groups. Preferred are aralkyl groups having from 7 to 10 carbon atoms, particularly aralkyl groups having from 7 to 9 carbon atoms. Most preferred is the α,α-dimethylphenylmethyl (cumyl) group.

It is preferred that Y represents an oxygen atom.

The nitrogen atom of the piperidine ring of the compounds of formula (I) can readily form acid addition salts with various acids. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid. Specifically preferred acids are selected from: fumaric acid, tartaric acid, succinic acid, citric acid, oxalic acid, lactic acid, maleic acid, methanesulphonic acid, trifluoromethanesulphonic acid, ethanesulphonic acid, benzenesulphonic acid, p-toluenesulphonic acid, hydrochloric acid, hydrobromic acid, hydrofluoric acid, hydroiodic acid, sulphuric acid and phosphoric acid.

It will be appreciated that other derivatives of the compounds of formula (I) will also be able to form acid addition salts, where appropriate, and that suitable acids are as described above.

The compounds of the present invention necessarily contain at least one asymmetric carbon atom in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

Particularly preferred meanings for the substituents of the compounds of formula (I) are set out below.

R preferably represents an alkanedioyl group having from 4 to 12 carbon atoms, an alkanoyl group having from 2 to 4 carbon atoms, or a benzoyl group which is unsubstituted or which is substituted with 1 or 2 alkyl groups having from 1 to 4 carbon atoms;

$R^1$ preferably represents a hydrogen atom or an alkanoyl group having from 2 to 6 carbon atoms, a haloformyl group or a benzoyl group which is unsubstituted or which is substituted with 1 or 2 alkyl groups having from 1 to 4 carbon atoms;

$R^2$ represents a phenyl group which is unsubstituted or which has 1 to 3 substituents selected from
an alkyl group having from 1 to 4 carbon atoms, an
aralkyl group having from 7 to 9 carbon atoms, a
halomethyl group and a hydroxyl group,

a benzoyl group which is unsubstituted or which is substituted with 1 to 3 alkyl groups each having from 1 to 4 carbon atoms, or an arylalkanoyl group having 9 carbon atoms and which is unsubstituted or which has 1 to 3 substituents selected from alkyl groups having from 1 to 4 carbon atoms and hydroxyl groups;

Y represents an oxygen atom; and

n is 1 or 2.

Most preferred meanings are as follows:

R represents an alkanedioyl group having from 8 to 10 carbon atoms;

$R^1$ represents a hydrogen atom or an acetyl group;

$R^2$ represents a phenyl group which is unsubstituted or which has 1 to 3 substituents selected from alkyl groups having from 1 to 4 carbon atoms and aralkyl groups having from 7 to 9 carbon atoms; and

n is 2.

Typical examples of the compounds of the general formula (I) are shown in Table 1.

In the Table, the following abbreviations are used to refer to certain substituent groups:

| | |
|---|---|
| Ac | acetyl |
| All | allyl |
| Boz | benzoyl |
| Bu | butyl |

| | |
|---|---|
| Bun | butenyl |
| Byr | butyryl |
| Bz | benzyl |
| Cum | cumyl |
| Et | ethyl |
| Furo | furoyl |
| Laur | lauroyl |
| Me | methyl |
| Nic | nicotinyl |
| Ph | phenyl |
| Phtl | phthaloyl |
| Pr | propyl |
| Prn | propionyl |
| Ste | stearoyl |
| tPhtl | terephthaloyl |
| Thno | thenoyl |
| Tol | tolyl |
| Tolo | toluoyl |
| Va | valeryl |
| Xyl | xylyl |
| Xylo | xyloyl |
| i | iso |
| t | tertiary |
| c | cyclo |

Table 1

| Cmpd No. | R | n | $R^1$ | Y | $R^2$ |
|---|---|---|---|---|---|
| 1 | H | 1 | H | O | Me |
| 2 | H | 1 | H | O | Ph |
| 3 | Ac | 1 | H | O | Me |
| 4 | Ac | 1 | Ac | O | Me |
| 5 | Ac | 1 | H | O | Ph |
| 6 | Ac | 1 | Ac | O | Ph |
| 7 | Pr | 1 | H | O | Ph |
| 8 | Pr | 1 | Ac | O | Ph |
| 9 | Byr | 1 | H | O | Ph |
| 10 | Byr | 1 | Ac | O | Ph |
| 11 | Va | 1 | H | O | Ph |
| 12 | Va | 1 | Ac | O | Ph |
| 13 | Laur | 1 | H | O | Ph |
| 14 | Laur | 1 | Laur | O | Laur |
| 15 | Ste | 1 | H | O | Ph |
| 16 | Ste | 1 | Ste | O | Ste |
| 17 | Boz | 1 | H | O | Me |
| 18 | Boz | 1 | Ac | O | Me |
| 19 | Boz | 1 | H | O | All |
| 20 | Boz | 1 | Ac | O | All |
| 21 | Boz | 1 | H | O | Ph |
| 22 | Boz | 1 | Ac | O | Ph |
| 23 | Boz | 1 | Boz | O | Ph |
| 24 | Boz | 1 | 2,4-Xylo | O | Ph |
| 25 | Boz | 1 | H | O | 2-Tol |
| 26 | Boz | 1 | Ac | O | 2-Tol |
| 27 | Boz | 1 | H | O | 2,6-Xyl |
| 28 | Boz | 1 | Ac | O | 2,6-Xyl |

Table 1 (cont'd)

| Cmpd No. | R | n | $R^1$ | Y | $R^2$ |
|---|---|---|---|---|---|
| 29 | Boz | 1 | H | O | 2-tBuPh |
| 30 | Boz | 1 | Ac | O | 2-tBuPh |
| 31 | Boz | 1 | H | O | 4-tBuPh |
| 32 | Boz | 1 | Ac | O | 4-tBuPh |
| 33 | Boz | 1 | 4-tBuBoz | O | 4-tBuPh |
| 34 | Boz | 1 | H | O | 2-tBu-6-MePh |
| 35 | Boz | 1 | Ac | O | 2-tBu-6-MePh |
| 36 | Boz | 1 | H | O | 3,5-di(tBu)-4-OH-Ph |
| 37 | Boz | 1 | Ac | O | 3,5-di(tBu)-4-OH-Ph |
| 38 | Boz | 1 | H | O | 4-CumPh |
| 39 | Boz | 1 | Ac | O | 4-CumPh |
| 40 | Boz | 1 | H | O | Ac |
| 41 | Boz | 1 | Ac | O | Ac |
| 42 | Boz | 1 | H | O | $CF_3CO$- |
| 43 | Boz | 1 | $CF_3CO$- | O | $CF_3CO$- |
| 44 | Boz | 1 | H | O | Boz |
| 45 | Boz | 1 | Boz | O | Boz |
| 46 | Boz | 1 | H | O | 2-Tolo |
| 47 | Boz | 1 | 2-Tolo | O | 2-Tolo |
| 48 | Boz | 1 | H | O | 4-tBuBoz |
| 49 | Boz | 1 | Ac | O | 4-tBuBoz |
| 50 | 4-tBuBoz | 1 | H | O | 4-tBuBoz |
| 51 | 4-tBuBoz | 1 | 4-tBuBoz | O | 4-tBuBoz |
| 52 | Boz | 1 | H | O | 3-[3,5-di(tBu)-4-OHPh]Prn |
| 53 | Boz | 1 | Ac | O | 3-[3-tBu-5-Me-4-OHPh]Prn |
| 54 | Boz | 1 | H | S | Ph |
| 55 | Boz | 1 | Ac | S | Ph |
| 56 | Boz | 1 | H | S | 4-tBuPh |

Table 1 (cont'd)

| Cmpd No. | R | n | $R^1$ | Y | $R^2$ |
|---|---|---|---|---|---|
| 57 | Boz | 1 | Ac | S | 4-tBuPh |
| 58 | 2-Thno | 1 | H | O | Ph |
| 59 | Nic | 1 | H | O | Ph |
| 60 | iNic | 1 | H | O | Ph |
| 61 | 2-Furo | 1 | H | O | Ph |
| 62 | $CH_2(CO-)_2$ | 2 | H | O | Me |
| 63 | $CH_2(CO-)_2$ | 2 | H | O | Ph |
| 64 | $CH_2(CO-)_2$ | 2 | H | O | 4-tBuPh |
| 65 | $CH_2(CO-)_2$ | 2 | Ac | O | 4-tBuPh |
| 66 | $CH_2(CO-)_2$ | 2 | H | O | 4-CumPh |
| 67 | $CH_2(CO-)_2$ | 2 | Ac | O | 4-CumPh |
| 68 | $CH_2(CO-)_2$ | 2 | H | O | Ac |
| 69 | $CH_2(CO-)_2$ | 2 | Ac | O | Ac |
| 70 | $(CH_2)_2(CO-)_2$ | 2 | H | O | Me |
| 71 | $(CH_2)_2(CO-)_2$ | 2 | Ac | O | Me |
| 72 | $(CH_2)_2(CO-)_2$ | 2 | Ac | O | Pr |
| 73 | $(CH_2)_2(CO-)_2$ | 2 | Ac | O | Pr |
| 74 | $(CH_2)_2(CO-)_2$ | 2 | H | O | Ph |
| 75 | $(CH_2)_2(CO-)_2$ | 2 | Ac | O | Ph |
| 76 | $(CH_2)_2(CO-)_2$ | 2 | H | O | 2-tBuPh |
| 77 | $(CH_2)_2(CO-)_2$ | 2 | Ac | O | 2-tBuPh |
| 78 | $(CH_2)_2(CO-)_2$ | 2 | H | O | 4-tBuPh |
| 79 | $(CH_2)_2(CO-)_2$ | 2 | Ac | O | 4-tBuPh |
| 80 | $(CH_2)_2(CO-)_2$ | 2 | H | O | 4-CumPh |
| 81 | $(CH_2)_2(CO-)_2$ | 2 | Ac | O | 4-CumPh |
| 82 | $(CH_2)_2(CO-)_2$ | 2 | H | O | 2,4-Xylo |
| 83 | $(CH_2)_2(CO-)_2$ | 2 | Ac | O | 2,4-Xylo |
| 84 | $(CH_2)_4(CO-)_2$ | 2 | H | O | Et |

Table 1 (cont'd)

| Cmpd No. | R | n | $R^1$ | Y | $R^2$ |
|---|---|---|---|---|---|
| 85 | $(CH_2)_4(CO-)_2$ | 2 | Ac | O | Et |
| 86 | $(CH_2)_4(CO-)_2$ | 2 | H | O | Ph |
| 87 | $(CH_2)_4(CO-)_2$ | 2 | Ac | O | Ph |
| 88 | $(CH_2)_4(CO-)_2$ | 2 | H | O | 2-tBuPh |
| 89 | $(CH_2)_4(CO-)_2$ | 2 | Ac | O | 2-tBuPh |
| 90 | $(CH_2)_4(CO-)_2$ | 2 | H | O | 4-tBuPh |
| 91 | $(CH_2)_4(CO-)_2$ | 2 | Ac | O | 4-tBuPh |
| 92 | $(CH_2)_4(CO-)_2$ | 2 | H | O | Ac |
| 93 | $(CH_2)_4(CO-)_2$ | 2 | Ac | O | Ac |
| 94 | $(CH_2)_4(CO-)_2$ | 2 | H | O | $CF_3CO-$ |
| 95 | $(CH_2)_4(CO-)_2$ | 2 | Ac | O | $CF_3CO-$ |
| 96 | $(CH_2)_4(CO-)_2$ | 2 | Ac | O | 4-tBuBoz |
| 97 | $(CH_2)_4(CO-)_2$ | 2 | $CF_3CO$ | O | 4-tBuBoz |
| 98 | $(CH_2)_5(CO-)_2$ | 2 | H | O | tBu |
| 99 | $(CH_2)_5(CO-)_2$ | 2 | Ac | O | tBu |
| 100 | $(CH_2)_5(CO-)_2$ | 2 | H | O | Ph |
| 101 | $(CH_2)_5(CO-)_2$ | 2 | Ac | O | Ph |
| 102 | $(CH_2)_5(CO-)_2$ | 2 | H | O | 2-Tol |
| 103 | $(CH_2)_5(CO-)_2$ | 2 | Ac | O | 2-Tol |
| 104 | $(CH_2)_5(CO-)_2$ | 2 | H | O | 4-tBuPh |
| 105 | $(CH_2)_5(CO-)_2$ | 2 | Ac | O | 4-tBuPh |
| 106 | $(CH_2)_5(CO-)_2$ | 2 | H | O | 2-tBu-6-MePh |
| 107 | $(CH_2)_5(CO-)_2$ | 2 | Ac | O | 2-tBu-6-MePh |
| 108 | $(CH_2)_5(CO-)_2$ | 2 | H | O | 2-Tolo |
| 109 | $(CH_2)_5(CO-)_2$ | 2 | Ac | O | 2-Tolo |
| 110 | $(CH_2)_5(CO-)_2$ | 2 | Ac | O | 3-[3,5-di(tBu)-4-OHPh]Prn |
| 111 | $(CH_2)_5(CO-)_2$ | 2 | Ac | O | 3-[3,5-di(tBu)-4-OHPh]Prn |
| 112 | $(CH_2)_6(CO-)_2$ | 2 | H | O | Ph |

Table 1 (cont'd)

| Cmpd No. | R | n | $R^1$ | Y | $R^2$ |
|---|---|---|---|---|---|
| 113 | $(CH_2)_6(CO-)_2$ | 2 | Ac | O | Ph |
| 114 | $(CH_2)_6(CO-)_2$ | 2 | H | O | 2,6-Xyl |
| 115 | $(CH_2)_6(CO-)_2$ | 2 | Ac | O | 2,6-Xyl |
| 116 | $(CH_2)_6(CO-)_2$ | 2 | H | O | 4-tBuPh |
| 117 | $(CH_2)_6(CO-)_2$ | 2 | Ac | O | 4-tBuPh |
| 118 | $(CH_2)_6(CO-)_2$ | 2 | H | O | Ac |
| 119 | $(CH_2)_6(CO-)_2$ | 2 | Ac | O | Ac |
| 120 | $(CH_2)_6(CO-)_2$ | 2 | H | O | Boz |
| 121 | $(CH_2)_7(CO-)_2$ | 2 | H | O | Me |
| 122 | $(CH_2)_7(CO-)_2$ | 2 | Ac | O | Me |
| 123 | $(CH_2)_7(CO-)_2$ | 2 | H | O | Ph |
| 124 | $(CH_2)_7(CO-)_2$ | 2 | Ac | O | Ph |
| 125 | $(CH_2)_7(CO-)_2$ | 2 | H | O | 2,6-Xyl |
| 126 | $(CH_2)_7(CO-)_2$ | 2 | Ac | O | 2,6-Xyl |
| 127 | $(CH_2)_7(CO-)_2$ | 2 | H | O | 4-tBuPh |
| 128 | $(CH_2)_7(CO-)_2$ | 2 | Ac | O | 4-tBuPh |
| 129 | $(CH_2)_7(CO-)_2$ | 2 | H | O | 3,5-di(tBu)-4-OH-Ph |
| 130 | $(CH_2)_7(CO-)_2$ | 2 | Ac | O | 3,5-di(tBu)-4-OH-Ph |
| 131 | $(CH_2)_7(CO-)_2$ | 2 | H | O | 4-CumPh |
| 132 | $(CH_2)_7(CO-)_2$ | 2 | Ac | O | 4-CumPh |
| 133 | $(CH_2)_7(CO-)_2$ | 2 | H | O | Ac |
| 134 | $(CH_2)_7(CO-)_2$ | 2 | Ac | O | Ac |
| 135 | $(CH_2)_7(CO-)_2$ | 2 | H | O | 2,6-Xylo |
| 136 | $(CH_2)_7(CO-)_2$ | 2 | Ac | O | 2,6-Xylo |
| 137 | $(CH_2)_8(CO-)_2$ | 2 | H | O | Ph |
| 138 | $(CH_2)_8(CO-)_2$ | 2 | Ac | O | Ph |
| 139 | $(CH_2)_8(CO-)_2$ | 2 | Boz | O | Ph |
| 140 | $(CH_2)_8(CO-)_2$ | 2 | 2,6-Xylo | O | Ph |

Table 1 (cont'd)

| Cmpd No. | R | n | $R^1$ | Y | $R^2$ |
|---|---|---|---|---|---|
| 141 | $(CH_2)_8(CO-)_2$ | 2 | 4-tBuBoz | O | Ph |
| 142 | $(CH_2)_8(CO-)_2$ | 2 | H | O | 2-tBuPh |
| 143 | $(CH_2)_8(CO-)_2$ | 2 | Ac | O | 2-tBuPh |
| 144 | $(CH_2)_8(CO-)_2$ | 2 | H | O | 4-tBuPh |
| 145 | $(CH_2)_8(CO-)_2$ | 2 | Ac | O | 4-tBuPh |
| 146 | $(CH_2)_8(CO-)_2$ | 2 | H | O | 2-tBu-6-MePh |
| 147 | $(CH_2)_8(CO-)_2$ | 2 | Ac | O | 2-tBu-6-MePh |
| 148 | $(CH_2)_8(CO-)_2$ | 2 | H | O | 3,5-di(tBu)-4-OH-Ph |
| 149 | $(CH_2)_8(CO-)_2$ | 2 | Ac | O | 3,5-di(tBu)-4-OH-Ph |
| 150 | $(CH_2)_8(CO-)_2$ | 2 | H | O | Ac |
| 151 | $(CH_2)_8(CO-)_2$ | 2 | Ac | O | Ac |
| 152 | $(CH_2)_8(CO-)_2$ | 2 | H | O | Boz |
| 153 | $(CH_2)_8(CO-)_2$ | 2 | Ac | O | Boz |
| 154 | $(CH_2)_8(CO-)_2$ | 2 | H | O | 4-tBuBoz |
| 155 | $(CH_2)_8(CO-)_2$ | 2 | Ac | O | 4-tBuBoz |
| 156 | $(CH_2)_8(CO-)_2$ | 2 | H | O | 3-PhPrn |
| 157 | $(CH_2)_8(CO-)_2$ | 2 | Ac | O | 3-PhPrn |
| 158 | $(CH_2)_9(CO-)_2$ | 2 | H | O | Et |
| 159 | $(CH_2)_9(CO-)_2$ | 2 | Ac | O | Et |
| 160 | $(CH_2)_9(CO-)_2$ | 2 | H | O | Bun |
| 161 | $(CH_2)_9(CO-)_2$ | 2 | Ac | O | Bun |
| 162 | $(CH_2)_9(CO-)_2$ | 2 | H | O | Ph |
| 163 | $(CH_2)_9(CO-)_2$ | 2 | Ac | O | Ph |
| 164 | $(CH_2)_9(CO-)_2$ | 2 | H | O | 2,6-Xyl |
| 165 | $(CH_2)_9(CO-)_2$ | 2 | Ac | O | 2,6-Xyl |
| 166 | $(CH_2)_9(CO-)_2$ | 2 | H | O | 2-tBuPh |
| 167 | $(CH_2)_9(CO-)_2$ | 2 | Ac | O | 2-tBuPh |
| 168 | $(CH_2)_9(CO-)_2$ | 2 | H | O | 4-tBuPh |

Table 1 (cont'd)

| Cmpd No. | R | n | $R^1$ | Y | $R^2$ |
|---|---|---|---|---|---|
| 169 | $(CH_2)_9(CO-)_2$ | 2 | Ac | O | 4-tBuPh |
| 170 | $(CH_2)_9(CO-)_2$ | 2 | H | O | 2-tBu-6-MePh |
| 171 | $(CH_2)_9(CO-)_2$ | 2 | Ac | O | 2-tBu-6-MePh |
| 172 | $(CH_2)_9(CO-)_2$ | 2 | H | O | 3,5-di(tBu)-4-OH-Ph |
| 173 | $(CH_2)_9(CO-)_2$ | 2 | Ac | O | 3,5-di(tBu)-4-OH-Ph |
| 174 | $(CH_2)_9(CO-)_2$ | 2 | H | O | 4-CumPh |
| 175 | $(CH_2)_9(CO-)_2$ | 2 | Ac | O | 4-CumPh |
| 176 | $(CH_2)_9(CO-)_2$ | 2 | H | O | PhAc |
| 177 | $(CH_2)_9(CO-)_2$ | 2 | Ac | O | PhAc |
| 178 | $(CH_2)_9(CO-)_2$ | 2 | H | O | Boz |
| 179 | $(CH_2)_9(CO-)_2$ | 2 | Ac | O | Boz |
| 180 | $(CH_2)_9(CO-)_2$ | 2 | H | O | 4-tBuBoz |
| 181 | $(CH_2)_9(CO-)_2$ | 2 | Ac | O | 4-tBuBoz |
| 182 | $(CH_2)_9(CO-)_2$ | 2 | 2,4-Xylo | O | 4-tBuBoz |
| 183 | $(CH_2)_9(CO-)_2$ | 2 | 4-tBuBoz | O | 4-tBuBoz |
| 184 | $(CH_2)_{10}(CO-)_2$ | 2 | H | O | Me |
| 185 | $(CH_2)_{10}(CO-)_2$ | 2 | H | O | All |
| 186 | $(CH_2)_{10}(CO-)_2$ | 2 | Ac | O | All |
| 187 | $(CH_2)_{10}(CO-)_2$ | 2 | H | O | Bz |
| 188 | $(CH_2)_{10}(CO-)_2$ | 2 | Ac | O | Bz |
| 189 | $(CH_2)_{10}(CO-)_2$ | 2 | H | O | Ph |
| 190 | $(CH_2)_{10}(CO-)_2$ | 2 | Ac | O | Ph |
| 191 | $(CH_2)_{10}(CO-)_2$ | 2 | $CF_3CO$ | O | Ph |
| 192 | $(CH_2)_{10}(CO-)_2$ | 2 | H | O | 4-tBuPh |
| 193 | $(CH_2)_{10}(CO-)_2$ | 2 | Ac | O | 4-tBuPh |
| 194 | $(CH_2)_{10}(CO-)_2$ | 2 | $CF_3CO$ | O | 4-tBuPh |
| 195 | $(CH_2)_{10}(CO-)_2$ | 2 | $PhCH_2CO$ | O | 4-tBuPh |
| 196 | $(CH_2)_{10}(CO-)_2$ | 2 | H | O | Ac |

Table 1 (cont'd)

| Cmpd No. | R | n | $R^1$ | Y | $R^2$ |
|---|---|---|---|---|---|
| 197 | $(CH_2)_{10}(CO-)_2$ | 2 | Ac | O | Ac |
| 198 | $(CH_2)_{10}(CO-)_2$ | 2 | H | O | $CF_3CO-$ |
| 199 | $(CH_2)_{10}(CO-)_2$ | 2 | Ac | O | $CF_3CO-$ |
| 200 | $(CH_2)_{10}(CO-)_2$ | 2 | H | O | Boz |
| 201 | $(CH_2)_{10}(CO-)_2$ | 2 | Ac | O | Boz |
| 202 | $(CH_2)_{10}(CO-)_2$ | 2 | H | O | 2-Tolo |
| 203 | $(CH_2)_{10}(CO-)_2$ | 2 | Ac | O | 2-Tolo |
| 204 | $(CH_2)_{10}(CO-)_2$ | 2 | H | O | PhAc |
| 205 | $(CH_2)_{10}(CO-)_2$ | 2 | Ac | O | PhAc |
| 206 | $(CH_2)_{10}(CO-)_2$ | 2 | H | O | 3-[3,5-di(tBu)-4-OHPh]Prn |
| 207 | $(CH_2)_{10}(CO-)_2$ | 2 | Ac | O | 3-[3,5-di(tBu)-4-OHPh]Prn |
| 208 | $(CH_2)_{11}(CO-)_2$ | 2 | H | O | Me |
| 209 | $(CH_2)_{11}(CO-)_2$ | 2 | Ac | O | Me |
| 210 | $(CH_2)_{11}(CO-)_2$ | 2 | H | O | All |
| 211 | $(CH_2)_{11}(CO-)_2$ | 2 | Ac | O | All |
| 212 | $(CH_2)_{11}(CO-)_2$ | 2 | H | O | Bz |
| 213 | $(CH_2)_{11}(CO-)_2$ | 2 | Ac | O | Bz |
| 214 | $(CH_2)_{11}(CO-)_2$ | 2 | H | O | Ph |
| 215 | $(CH_2)_{11}(CO-)_2$ | 2 | Ac | O | Ph |
| 216 | $(CH_2)_{11}(CO-)_2$ | 2 | H | O | 4-tBu-Ph |
| 217 | $(CH_2)_{11}(CO-)_2$ | 2 | Ac | O | 4-tBu-Ph |
| 218 | $(CH_2)_{11}(CO-)_2$ | 2 | H | O | Ac |
| 219 | $(CH_2)_{11}(CO-)_2$ | 2 | Ac | O | Ac |
| 220 | $(CH_2)_{11}(CO-)_2$ | 2 | H | O | $CF_3CO-$ |
| 221 | $(CH_2)_{11}(CO-)_2$ | 2 | Ac | O | $CF_3CO-$ |
| 222 | $(CH_2)_{11}(CO-)_2$ | 2 | H | O | Boz |

Table 1 (cont'd)

| Cmpd No. | R | n | $R^1$ | Y | $R^2$ |
|---|---|---|---|---|---|
| 223 | $(CH_2)_{11}(CO-)_2$ | 2 | Ac | O | Boz |
| 224 | $(CH_2)_{11}(CO-)_2$ | 2 | Boz | O | Boz |
| 225 | $(CH_2)_{11}(CO-)_2$ | 2 | 2,6-Xylo | O | Boz |
| 226 | $(CH_2)_{11}(CO-)_2$ | 2 | 4-tBuBoz | O | Boz |
| 227 | $(CH_2)_{11}(CO-)_2$ | 2 | H | O | 2-Tolo |
| 228 | $(CH_2)_{11}(CO-)_2$ | 2 | Ac | O | 2-Tolo |
| 229 | $(CH_2)_{11}(CO-)_2$ | 2 | H | O | PhAc |
| 230 | $(CH_2)_{11}(CO-)_2$ | 2 | Ac | O | PhAc |
| 231 | $(CH_2)_{11}(CO-)_2$ | 2 | H | O | Me |
| 232 | $(CH_2)_{11}(CO-)_2$ | 2 | Ac | O | Me |
| 233 | $(CH_2)_{11}(CO-)_2$ | 2 | H | O | All |
| 234 | $(CH_2)_{11}(CO-)_2$ | 2 | Ac | O | All |
| 235 | $(CH_2)_{11}(CO-)_2$ | 2 | H | O | Bz |
| 236 | $(CH_2)_{11}(CO-)_2$ | 2 | Ac | O | Bz |
| 237 | $(CH_2)_{11}(CO-)_2$ | 2 | H | O | Ph |
| 238 | $(CH_2)_{11}(CO-)_2$ | 2 | Ac | O | Ph |
| 239 | $(CH_2)_{11}(CO-)_2$ | 2 | H | O | 2-tBuPh |
| 240 | $(CH_2)_{11}(CO-)_2$ | 2 | Ac | O | 2-tBuPh |
| 241 | $(CH_2)_{11}(CO-)_2$ | 2 | H | O | 4-tBuPh |
| 242 | $(CH_2)_{11}(CO-)_2$ | 2 | Ac | O | 4-tBuPh |
| 243 | $(CH_2)_{11}(CO-)_2$ | 2 | H | O | 3,5-di(tBu)-4-OH-Ph |
| 244 | $(CH_2)_{11}(CO-)_2$ | 2 | Ac | O | 3,5-di(tBu)-4-OH-Ph |
| 245 | $(CH_2)_{11}(CO-)_2$ | 2 | H | O | Ac |
| 246 | $(CH_2)_{11}(CO-)_2$ | 2 | Ac | O | Ac |
| 247 | $(CH_2)_{11}(CO-)_2$ | 2 | H | O | $CF_3CO-$ |
| 248 | $(CH_2)_{11}(CO-)_2$ | 2 | Ac | O | $CF_3CO-$ |
| 249 | $(CH_2)_{11}(CO-)_2$ | 2 | H | O | Boz |
| 250 | $(CH_2)_{11}(CO-)_2$ | 2 | H | O | Boz |

Table 1 (cont'd)

| Cmpd No. | R | n | $R^1$ | Y | $R^2$ |
|---|---|---|---|---|---|
| 251 | $(CH_2)_{11}(CO-)_2$ | 2 | H | O | 2-Tolo |
| 252 | $(CH_2)_{11}(CO-)_2$ | 2 | Ac | O | 2-Tolo |
| 253 | $(CH_2)_{11}(CO-)_2$ | 2 | H | O | PhAc |
| 254 | $(CH_2)_{11}(CO-)_2$ | 2 | Ac | O | PhAc |
| 255 | $(CH_2)_8(CO-)_2$ | 2 | H | S | Ph |
| 256 | $(CH_2)_8(CO-)_2$ | 2 | Ac | S | Ph |
| 257 | $(CH_2)_8(CO-)_2$ | 2 | H | S | 2-tBuPh |
| 258 | $(CH_2)_8(CO-)_2$ | 2 | Ac | S | 2-tBuPh |
| 259 | $(CH_2)_8(CO-)_2$ | 2 | $CF_3CO$ | S | 2-tBuPh |
| 260 | $(CH_2)_8(CO-)_2$ | 2 | H | S | 4-tBuPh |
| 261 | $(CH_2)_8(CO-)_2$ | 2 | Ac | S | 4-tBuPh |
| 262 | $(CH_2)_8(CO-)_2$ | 2 | $CF_3CO$ | S | 4-tBuPh |
| 263 | Phtl | 2 | H | O | Ph |
| 264 | Phtl | 2 | Ac | O | Ph |
| 265 | iPhtl | 2 | H | O | Ph |
| 266 | iPhtl | 2 | Ac | O | Ph |
| 267 | tPhtl | 2 | H | O | Ph |
| 268 | tPhtl | 2 | Ac | O | Ph |
| 269 | 1,3,5-tri(CO-)Ph | 3 | H | O | Ph |
| 270 | 1,3,5-tri(CO-)Ph | 3 | Ac | O | Ph |
| 271 | 1,2,5,6-tetra(CO-)Ph | 4 | H | O | Ph |
| 272 | 1,2,5,6-tetra(CO-)Ph | 4 | Ac | O | Ph |
| 273 | 1,2-di(CO-)cHx | 2 | H | O | Ph |
| 274 | 1,2-di(CO-)cHx | 2 | Ac | O | Ph |
| 275 | 1,2,3-tri(CO-)Prn | 3 | H | O | Ph |
| 276 | 1,2,3-tri(CO-)Prn | 3 | Ac | O | Ph |
| 277 | 1,2,3,4-tetra(CO-)Bu | 4 | H | O | Ph |
| 278 | 1,2,3,4-tetra(CO-)Bu | 4 | Ac | O | Ph |

Of the compounds listed above, the following are preferred, that is to say Compounds No. 3, 4, 5, 6, 7, 8, 9, 10, 17, 18, 21, 22, 23, 24, 25, 26, 27, 28, 29, 30, 31, 32, 33, 34, 35, 36, 37, 38, 39, 44, 45, 46, 47, 48, 49,

50, 51, 52, 53, 74, 75, 76, 77, 78, 79, 80, 81, 82, 83, 86, 87, 88, 89, 90, 91, 96, 97, 100, 101, 102, 103, 104, 105, 106, 107, 108, 109, 110, 111, 112, 113, 114, 115, 116, 117, 120, 123, 124, 125, 126, 127, 128, 129, 130, 131, 132, 135, 136, 137, 138, 139, 140, 141, 142, 143, 144, 145, 146, 147, 148, 149, 152, 153, 154, 155, 156, 157, 162, 163, 164, 165, 166, 167, 168, 169, 170, 171, 172, 173, 174, 175, 176, 177, 178, 179, 180, 181, 182, 183, 189, 190, 191, 192, 193, 194, 195, 200, 201, 202, 203, 204, 205, 206 and 207, of which Compounds No. 112, 113, 114, 115, 116, 117, 120, 123, 124, 125, 126, 127, 128, 129, 130, 131, 132, 135, 136, 137, 138, 139, 140, 141, 142, 143, 144, 145, 146, 147, 148, 149, 152, 153, 154, 155, 156 and 157 are more preferred.

The most preferred compounds of the present invention are Compounds No. 137 and 144, that is to say:

137. Di[2,2,6,6-tetramethyl-1-(2-hydroxy-3-phenoxypropyl)-4-piperidyl] sebacate and

144. Di{2,2,6,6-tetramethyl-1-[2-hydroxy-3-(4-t-butylphenoxy)propyl]-4-piperidyl} sebacate.

The compounds of the present invention can be prepared by any suitable method generally known in the art for the preparation of compounds of this type. One suitable general process is as follows:

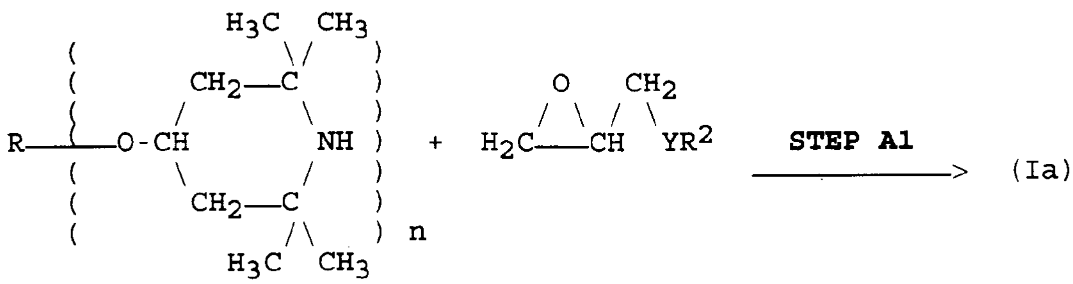


(II) (III)

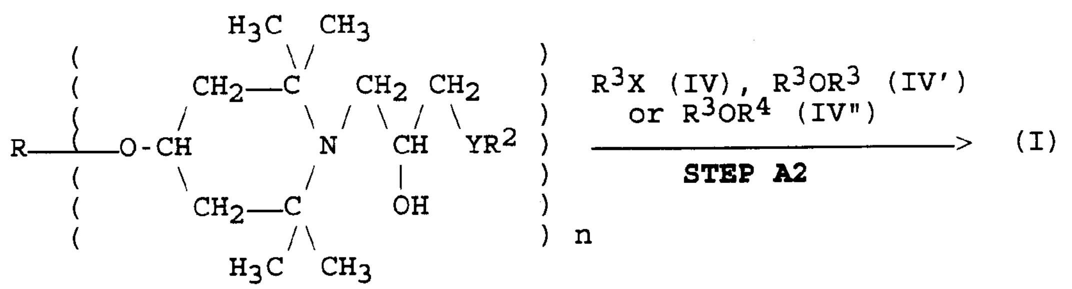


(Ia)

In the above reaction scheme, R, $R^1$, $R^2$, Y and n are as defined above, $R^3$ is as defined for $R^1$, but cannot represent a hydrogen atom, $R^4$ represents an alkyl group having from 1 to 4 carbon atoms, and X represents a halogen atom such as a fluorine, chlorine, bromine or iodine atom, preferably a chlorine or bromine atom.

In the reaction depicted in step A1, a compound of formula (Ia), which corresponds to a compound of formula (I), but wherein $R^1$ is hydrogen, is produced by an addition reaction between a compound of formula (II) and a compound of formula (III).

Step A1 may be performed in the presence or absence of a solvent, as desired, although it is generally preferred to perform this step in the absence of solvent. This Step may be also be performed in the presence or absence of an acid, as desired, but it is generally preferred to perform Step A1 in the presence of acid.

Compounds of formula (II) can be prepared, for example, by the process described in Japanese Patent Publication (Kokoku) Sho-56-32308 (US Patent No. 3840494, Murayama K., et al, published 8 October 1974). Compounds of formula (III) can be prepared, for example, as described in J.A.C.S. (1979), 101, 3666.

When a solvent is employed in the reaction of Step A1, then suitable solvents include: ethers, such as ethylene glycol dimethyl ether, ethylene glycol diethyl ether, diethyl ether, dioxane and tetrahydrofuran; halogenated hydrocarbons, such as methylene chloride, chloroform, carbon tetrachloride and dichloroethane; aromatic hydrocarbons, such as benzene, toluene and xylene; acetates, such as ethyl acetate and butyl acetate;

nitriles, such as acetonitrile; hydrocarbons, such as delete hexane, heptane, octane and nonane; alcohols such as methanol, ethanol, propanol, isopropanol and butanol; and mixed solvents of water and alcohols; preferably aromatic hydrocarbons. There is no particular restriction on the nature of the solvent to be employed, provided that it has no adverse effect on the reaction or on the reagents involved and that it can dissolve the reagents, at least to some extent.

Acids which may be employed in Step A1 include: sulphonic acids, such as benzenesulphonic acid, p-toluenesulphonic acid, trifluoromethanesulphonic acid and methanesulphonic acid; mineral acids, such as hydrochloric acid and sulphuric acid; and Lewis acids, such as anhydrous aluminum trichloride and boron trifluoride diethyl ether complex. The most preferred acids are Lewis acids.

The reaction can take place over a wide range of temperatures, and the precise reaction temperature is not critical to the invention. In general, we find it convenient to perform the reaction at a temperature between about 20 and about 200°C, preferably between about 80 and about 160°C, for a period of between about 10 and about 100 hours, preferably between about 20 and about 50 hours.

After the reaction has been allowed to go to completion, the target compound can be collected from the reaction mixture by known procedures. For example, if insolubles are present in the reaction mixture, then these can be removed, such as by filtration. Subsequently, water can be added to the reaction mixture, or the reaction mixture can be washed with an alkaline solution such as aqueous sodium hydrogencarbonate. The resulting mixture is then suitably extracted with a water-immiscible organic solvent, such as diethyl ether, and the organic layer dried over a drying agent, such as anhydrous magnesium sulphate, after which the solvent can be removed by evaporation to give the target compound.

If desired, the target compound can be further purified, such as by recrystallisation or column chromatography.

If it is desired to obtain a compound of formula (I), wherein $R^1$ represents a group other than hydrogen, then the compound obtained in Step A1 can, if desired, be acylated by the procedure of Step A2.

In Step A2, a compound of formula (Ia) is reacted with an acylating agent having the general formula (IV), (IV') or (IV").

The acylating agent used in Step A2 may be any that is suitable, and appropriate acylating agents will be readily apparent to those skilled in the art. However, we prefer to use acylating agents selected from: acid halides, such as acetyl chloride, propionyl chloride and benzoyl chloride; acid anhydrides, such as acetic anhydride, trifluoroacetic anhydride and benzoic anhydride; and esters, such as ethyl acetate, ethyl propionate and ethyl benzoate. Most preferred are acid anhydrides.

If an acylating agent of formula (IV") is used, then the reaction is preferably carried out in a solvent in the presence of a strong base or a metal catalyst.

Suitable solvents for use with the acylating agent of formula (IV") include: aliphatic hydrocarbons, such as hexane, heptane, octane and isooctane; and aromatic hydrocarbons, such as benzene, toluene and xylene. Preferred are aromatic hydrocarbons, especially toluene and xylene. There is no particular restriction on the nature of the solvent to be employed, provided that it has no adverse effect on the reaction or on the reagents involved and that it can dissolve the reagents, at least to some extent.

Suitable bases for use with the acylating agent of formula (IV") include: alkali metal alkoxides, such as sodium methoxide, sodium ethoxide and potassium t-butoxide; and alkali metal amides, such as lithium amide, sodium amide and lithium isopropylamide. Preferred are alkali metal amides, especially lithium amide.

Suitable metal catalysts for use with the acylating agent of formula (IV") include: tetraalkyl titanate compounds, such as tetraisopropyl titanate; and tin compounds, such as dibutyltin oxide. Preferred are tetraalkyl titanate compounds, especially tetraisopropyl titanate.

The reaction can take place over a wide range of temperatures, and the precise reaction temperature is not critical to the invention. In general, we find it convenient to perform the reaction at a temperature between about 80 and about 180°C, preferably between about 100 and about 120°C, for a period of between about 5 and about 100 hours, preferably between about 10 and about 50 hours.

If an acylating agent of formula (IV) is used, then the reaction is preferably carried out in a solvent in the presence of a base.

Suitable solvents for use with the acylating agent of formula (IV) include: aliphatic hydrocarbons, such as hexane, heptane, octane and isooctane; aromatic hydrocarbons, such as benzene, toluene and xylene; ethers, such as diethyl ether, dioxane and tetrahydrofuran; halogenated hydrocarbons, such as methylene chloride, chloroform, carbon tetrachloride and dichloroethane. Preferred are halogenated hydrocarbons, especially methylene chloride. There is no particular restriction on the nature of the solvent to be employed, provided that it has no adverse effect on the reaction or on the reagents involved and that it can dissolve the reagents, at least to some extent.

Suitable bases for use with the acylating agent of formula (IV) include: alkali metal hydrogencarbonates,

such as sodium hydrogencarbonate and potassium hydrogencarbonate; alkali metal hydroxides, such as sodium hydroxide and potassium hydroxide; alkaline earth metal hydroxides, such as calcium hydroxide and magnesium hydroxide; alkali metal carbonates, such as sodium carbonate and potassium carbonate; alkali metal acetates, such as sodium acetate and potassium acetate; alkali metal fluorides, such as sodium fluoride and potassium fluoride; tertiary lower alkylamines, such as triethylamine, ethyldiisopropylamine and tributylamine; and tertiary cycloaliphatic amines, such as 1,8-diazabicyclo[5.4.0]undecan-7-ene and 1,4-diazabicyclo[2.2.2]octane. Preferred are tertiary lower alkylamines, especially triethylamine.

The reaction can take place over a wide range of temperatures, and the precise reaction temperature is not critical to the invention. In general, we find it convenient to perform the reaction at a temperature between about 0 and about 130°C, preferably between about 20 and about 100°C, for a period of between about 5 and about 100 hours, preferably between about 10 and about 50 hours.

If an acylating agent of formula (IV") is used, then the reaction is preferably carried out in a solvent.

Suitable solvents for use with the acylating agent of formula (IV") include: aromatic hydrocarbons, such as benzene, toluene and xylene; ethers, such as diethyl ether, dioxane and tetrahydrofuran; and halogenated hydrocarbons, such as methylene chloride, chloroform, carbon tetrachloride and dichloroethane. Preferred are halogenated hydrocarbons, especially methylene chloride. There is no particular restriction on the nature of the solvent to be employed, provided that it has no adverse effect on the reaction or on the reagents involved and that it can dissolve the reagents, at least to some extent.

If desired, then the acylating agent of formula (IV") can be used in great excess in order to act both as solvent and acylating agent, thereby avoiding any requirement for additional solvent.

The reaction can take place over a wide range of temperatures, and the precise reaction temperature is not critical to the invention. In general, we find it convenient to perform the reaction at a temperature between about 0 and about 130°C, preferably between about 20 and about 100°C, for a period of between about 5 and about 100 hours, preferably between about 10 and about 50 hours.

Once the acylation reaction of Step A2 has been allowed to proceed to completion, the target compound can be collected from the reaction mixture by conventional procedures. For example, if insolubles are present in the reaction mixture, then these can be removed, such as by filtration. Subsequently, water can be added to the reaction mixture, or the reaction mixture can be washed with an alkaline solution such as aqueous sodium hydrogencarbonate. The resulting mixture is then suitably extracted with a water-immiscible organic solvent, such as diethyl ether, and the organic layer dried over a drying agent, such as anhydrous magnesium sulphate, after which the solvent can be removed by evaporation to afford the target compound.

If desired, the target compound can be further purified, such as by recrystallisation or column chromatography.

The compounds of the present invention are suitable for use with appropriate polymers to increase photostability and heat stability.

The compounds of the present invention may be used in conjunction with polymers in any suitable fashion, and will generally be incorporated into the polymer prior to forming the final product.

Typically, the products which it is desired to stabilise will be moulded products, and the compounds of the present invention will be introduced into the polymer prior to moulding.

The stage at which the compounds of the invention are introduced, prior to moulding, is not critical, but it will be appreciated that it is desirable to achieve a substantially uniform distribution throughout the article to be moulded. Thus, it is preferred to introduce the compounds of the invention while the polymer, or its precursors, are in a liquid state, prior to setting.

The liquid state may be because the monomers, or polymer precursors, have yet to react sufficiently to form the final polymer, or it may be because, for example, the polymer itself has been melted, or is in solution, or both. The compound of the invention can be added at any of these stages.

Thus, the stabilisers of the present invention can readily be incorporated into polymers in any suitable, conventional fashion. For example, a stabiliser in the form of a dry powder can be mixed with the polymer, or a suspension or emulsion of stabiliser can be mixed with a polymer solution, suspension or emulsion.

Suitable synthetic polymers for use with the stabilisers of the present invention include:

<u>Olefins and diene polymers</u>

Homopolymers of olefins and dienes (e.g. low-density, linear low-density, high density and crosslinked polyethylenes, polypropylene, polyisobutene, polymethylbutene-1, polymethylpentene-1, polyisoprene and polybutadiene); mixtures of such homopolymers (e.g. mixtures of polypropylene and polyethylene, of polypropylene and polybutene-1 and of polypropylene and polyisobutylene); copolymers of olefins (e.g. ethylenepropylene copolymer, propylene-butene-1 copolymer and ethylene-butene-1 copolymer); and copolymers of olefins and dienes (e.g. terpolymers of ethylene, propylene and dienes, such as butadiene, hexadiene, dicyclopentadiene and ethylidene norbornene);

Styrene polymers

Copolymers of polystyrene, styrene or α-methylstyrene (e.g. styrene-maleic anhydride copolymer, styrene-butadiene copolymer, styrene-acrylonitrilemethyl methacrylate copolymer, styrene-acrylonitrileacrylate copolymer, styrene-acrylonitrile copolymer modified with an acrylate for impact strength, and styrene polymer modified with an ethylene propylene diene monomer, also for impact strength); and graft copolymers of styrene [e.g. a graft copolymer of styrene and polybutadiene, graft copolymers of styrene and acrylonitrile to polybutadiene (generally referred to as acrylonitrile-butadiene-styrene polymers) and mixtures of such graft copolymers with the styrene copolymers given above];

Vinyl halide and vinylidene halide polymers

Polyvinyl chloride, polyvinylidene chloride, polyvinylidene fluoride, polychloroprene, vinyl chloridevinylidene chloride copolymer, vinyl chloride-vinyl acetate copolymer, vinyl chloride-ethylene copolymer and vinylidene chloride-vinyl acetate copolymer;

Polymers of α,β-unsaturated acids and their derivatives

Polyacrylic acid esters, polymethacrylic acid esters, polyacrylamides and polyacrylonitriles;

Polymers of unsaturated alcohols and unsaturated amines or their acyl derivatives or acetals

Polyvinyl alcohols, polyvinyl acetate, polyvinyl stearate, polyvinyl benzoate, polyvinyl maleate, polyvinyl butyral, polyallyl phthalate or polyallyl melamine, and copolymers of such monomers with other vinyl compounds (e.g. ethylene-vinyl acetate copolymer);

Epoxy polymers

Homopolymers and copolymers of epoxy compounds (e.g. polyethylene oxide); and polymers of bisglycidyl ether compounds;

Polyacetals, polyalkylene oxides and polyphenylene oxides

Polyoxymethylene, oxymethylene-ethylene oxide copolymer, polyoxyethylene, polypropylene oxide, polyisobutylene oxide and polyphenylene oxide;

Polyurethanes and polyureas

Polycarbonates

Polysulfones

Polyamides and copolyamides

Polyamides and copolyamides derived from diamines and aliphatic acids or aromatic dicarboxylic acids and/or from aminocarboxylic acids or corresponding lactams (e.g. Nylon 6, Nylon 6/6, Nylon 6/10, Nylon 11 and Nylon 12);

Polyesters

Polyesters derived from dicarboxylic acids and dialcohols and/or from oxy acids or corresponding lactones (e.g. polyethylene terephthalate, polybutylene terephthalate, polycyclohexane-1,4-dimethylene terephthalate);

Cross-linked polymers

Cross-linked polymers consisting of a moiety derived from aldehydes and another moiety derived from phenol, urea or melamine (e.g. phenol formaldehyde resins, urea-formaldehyde resins, melamine-formaldehyde resins and diaryl phthalate resins);

Alkyd resins

Glycerol-phthalic acid resins and mixtures thereof with melamine-formaldehyde resins;

Unsaturated polyester resins

Unsaturated polyester resins derived from copolyesters of saturated and unsaturated dicarboxylic acids and polyhydric alcohols and prepared using vinyl compounds as cross-linking agents; and modified unsaturated polyester resins by chlorination for enhanced flame retardancy.

The quantity of stabiliser of the present invention necessary to achieve the desired stabilisation effect will vary depending on a number of factors which will be clear to those skilled in the art. However, typical factors which need to be taken into account include the type of polymer to be stabilised, its properties and its intended use, and especially whether other additives are to be used. In general, though, we prefer to use from about 0.01 to about 5% by weight of the stabiliser of the present invention based on the weight of the polymer.

The preferred amount used, as stated above, will vary depending on the kind of polymer. For example, for olefin, diene and styrene polymers, suitable amounts of stabiliser are from about 0.1 to about 2.0% by weight, preferably from about 0.02 to about 1.0% by weight; for vinyl chloride and vinylidene chloride polymers suitable amounts are from about 0.01 to about 1.0% by weight, preferably from about 0.02 to about 0.5% by weight; and for polyurethane and polyamide polymers, suitable amounts are from about 0.01 to about 5.0% by weight, preferably from about 0.02 to about 2.0% by weight.

It will be appreciated that two or more stabilisers of the present invention may be used in combination or with any other additives, as desired.

Various kinds of additives customarily used in the field of polymer technology can suitably be added separately or together with the stabilisers of the present invention. Suitable such additives include:

Phenol antioxidants

2,6-di-t-Butyl-p-cresol; stearyl-β-(4-hydroxy-3,5-di-t-butylphenyl) propionate; distearyl (4-hydroxy-3-methyl-5-t-butylbenzyl)malonate; 2,2'-methylenebis-(4-methyl-6-t-butylphenol); 4,4-methylenebis(2,6-di-t-butylphenol); 2,2'-methylenebis[6-(1-methylcyclohexyl) p-cresol]; bis[3,3-bis(4-hydroxy-3-t-butylphenyl)butyric acid] glycol ester; 4,4'-butylidenebis(6-t-butyl-m-cresol); 1,1,3-tris(2-methyl-4-hydroxy-5-t-butylphenyl)butane; 1,3,5-tris(3,5-di-t-butyl-4-hydroxy benzyl)-2,4,6-trimethylbenzene; 3,9-bis[1,1-dimethyl-2-(3,5-di-t-butyl-4-hydroxyphenyl)ethyl]-2,4,8,10-tetra-oxaspiro[5.5]undecane; pentaerythrityl tetrakis-[3-(3,5-di-t-butyl-4-hydroxyphenyl)propionate]; 1,3,5-tris(3,5-di-t-butyl-4-hydroxybenzyl)isocyanurate; 1,3,5-tris[(3,5-di-t-butyl-4-hydroxyphenyl)propionyloxyethyl]isocyanurate; and bis[3-(3,5-di-t-butyl-4-hydroxyphenyl)propionyl]oxamide.

Phosphite stabilizers

Tris(2,4-di-t-butylphenyl) phosphite, triphenyl phosphite; tris(nonylphenyl) phosphite; distearyl pentaerythritol diphosphite; 4,4-butylidenebis-(3-methyl-6-t-butylphenyl-di-tridecyl) phosphite; and bis(2,4-di-t-butylphenyl)pentaerythritol diphosphite.

Ultraviolet absorbers

2-Hydroxy-4-methoxybenzophenone; 2-hydroxy-4-octoxybenzophenone; 2, 4-dihydroxybenzophenone; 2-(2'-hydroxy-3'-t-butyl-5'-methylphenyl)-5-chlorobenzotriazole; 2-(2'-hydroxy-3',5'-di-t-butylphenyl)-5-chlorobenzotriazole; 2-(2'-hydroxy-5'-methylphenyl)benzotriazole; 2-(2'-hydroxy-3',5'-di-t-amylphenyl)benzotriazole; phenyl salicylate; and 2,4-di-t-butylphenyl-3,5-di-t-butyl-4-hydroxybenzoate.

Nickel-containing stabilizers

Nickel monoethyl-3,5-di-t-butyl-4-hydroxybenzyl-phosphonate; butylamine-nickel-2,2'-thiobis-(4-t-octylphenolate) complex; nickel dibutyl-dithiocarbamate; and nickel 3,5-di-t-butyl-4-hydroxybenzoate.

Metal salts of higher fatty acids

Calcium, magnesium, barium, zinc, cadmium, lead or nickel stearate, and calcium, magnesium, cadmium, barium or zinc laurate.

The stabilisers of the present invention may also be used in combination with such other agents as heavy metal deactivators, nucleating agents, organic tin compounds, plasticisers, epoxy compounds, pigments, fillers, foaming agents, antistatic agents, lubricants and processing aids.

Polymers incorporating the stabilisers of the present invention can be used as desired, such as in the form of films, fibers, tapes, compression moulding materials, coating compositions and paints.

Embodiments of the present invention will now be illustrated more fully by way of the accompanying Examples and Reference Examples. In the accompanying Examples and Reference Examples, "parts" and "%", where used, mean parts by weight and % by weight, respectively, unless otherwise specified.

## EXAMPLE 1

4-Benzoyloxy-2,2,6,6-tetramethyl-1-(2-hydroxy-3-phenoxypropyl)piperidine (Compound No. 21)

2.60 g (10 mmol) of 4-benzoyloxy-2,2,6,6-tetramethylpiperidine and 4.06 ml (30 mmol) of glycidyl phenyl ether were heated in a sealed tube in a 135°C oil bath for 50 hours. At the end of this time, the reaction mixture was concentrated by evaporation under reduced pressure to obtain crude crystals which were then recrystallised from ethyl acetate to afford 3.5 g (yield: 85%) of the target compound as colourless crystals.
Melting point: 124 - 125°C
Infrared Absorption Spectrum (KBr) $\nu_{max}$ $cm^{-1}$:
3250, 1710, 1285, 1118

| Elemental analysis: | | | |
|---|---|---|---|
| Calculated for $C_{25}H_{33}NO_4$ | | | |
| | C, 72.96%; | H, 8.08%; | N, 3.40%. |
| Found: | C, 73.64%; | H, 8.07%; | N, 3.42%. |

## EXAMPLE 2

4-Benzoyloxy-2,2,6,6-tetramethyl-1-(2-acetoxy-3-phenoxypropyl)piperidine (Compound No. 22)

1.50 g (3.64 mmol) of 4-benzoyloxy-2,2,6,6-tetramethyl-1-(2-hydroxy-3-phenoxypropyl)piperidine prepared as described in Example 1, 0.55 ml (4.00 mmol) of triethylamine and 0.38 ml (4.00 mmol) of acetic anhydride were mixed together in 10 ml of methylene chloride and heated under reflux for 15 hours. At the end of this time, a saturated aqueous solution of potassium carbonate was added to the reaction mixture, which was then extracted with methylene chloride, dried over anhydrous sodium sulphate and concentrated by evaporation under reduced pressure. The crude product thus obtained was purified by silica gel column chromatography (the eluent used was ethyl acetate hexane in a ratio of 1 : 10 by volume) to afford 1.34 g (yield: 81%) of the target compound as a viscous oil.
Infrared Absorption Spectrum (KBr) $\nu_{max}$ $cm^{-1}$:
1738, 1715, 1279, 1237, 1114

| Elemental analysis: | | | |
|---|---|---|---|
| Calculated for $C_{27}H_{35}NO_5$ | | | |
| | C, 71.50%; | H, 7.78%; | N, 3.09%. |
| Found: | C, 71.00%; | H, 7.90%; | N, 2.99%. |

EXAMPLE 3

Di[2,2,6,6-tetramethyl-1-(2-hydroxy-3-phenoxypropyl)-4-piperidyl] sebacate (Compound No. 137)

0.48 g (1.0 mmol) of di(2,2,6,6-tetramethyl-4-piperidyl) sebacate, and 0.54 ml (4.0 mmol) of glycidyl phenyl ether were mixed in 0.025 ml (0.20 mmol) of boron trifluoride diethyl ether complex and stirred at 100°C for 14 hours. At the end of this time, a saturated aqueous solution of sodium hydrogencarbonate was added to the reaction mixture, and the organic layer was extracted with ethyl acetate, dried over anhydrous sodium sulphate and concentrated by evaporation under reduced pressure. After excess glycidyl phenyl ether had been removed by further distillation under reduced pressure, the residue was recrystallised from a mixed solvent of acetone-hexane to afford 3.67 g (yield: 81%) of the target compound as colourless crystals.
Melting point: 86 - 89°C
Infrared Absorption Spectrum (KBr) $\nu_{max}$ $cm^{-1}$:
3440, 1730, 1245, 1174

| Elemental analysis: | | | |
|---|---|---|---|
| Calculated for $C_{46}H_{72}N_2O_8$ | | | |
| | C, 70.74%; | H, 9.29%; | N, 3.59%. |
| Found: | C, 70.48%; | H, 9.15%; | N, 3.67%. |

EXAMPLE 4

Di[2,2,6,6-tetramethyl-1-(2-acetoxy-3-phenoxypropyl)-4-piperidyl] sebacate (Compound No. 138)

Following a procedure similar to that of Example 2, 2.46 g (3.15 mmol) of di[2,2,6,6-tetramethyl-1-(2-hydroxy-3-phenoxypropyl)-4-piperidyl] sebacate, prepared as described in Example 3, was acetylated, and the crude product was purified by silica gel column chromatography (the eluent used was ethyl acetate : hexane in a ratio of 1 : 5 by volume) to afford 2.07 g (yield: 76.0%) of the target compound as a viscous oil.
Infrared Absorption Spectrum (neat) $\nu_{max}$ $cm^{-1}$:
1735, 1236, 1175

| Elemental analysis: | | | |
|---|---|---|---|
| Calculated for $C_{50}H_{76}N_2O_{10}$ | | | |
| | C, 69.41%; | H, 8.85%; | N, 3.24%. |
| Found: | C, 68.63%; | H, 9.04%; | N, 3.11%. |

EXAMPLE 5

4-Benzoyloxy-2,2,6,6-tetramethyl-1-(2-hydroxy-3-phenyl-thiopropyl)piperidine (Compound No. 54)

Following a procedure similar to that of Example 1, but using 2.16 g (10 mmol) of 4-benzoyloxy-2,2,6,6-tetramethylpiperidine and 4.99 g (30 mmol) glycidyl phenylthio ether prepared as described in Preparative Example 8, and purifying the product by silica gel column chromatography (the eluent used was ethyl acetate : hexane in a ratio of 1 : 10 by volume), the target compound was obtained as 2.81 g (yield: 65.8%) of a viscous oil.
Infrared Absorption Spectrum (neat) $\nu_{max}$ $cm^{-1}$:
3400, 1714, 1278, 1114

| Elemental analysis: | | | | |
|---|---|---|---|---|
| Calculated for $C_{33}H_{49}NO_5$ | | | | |
| | C, 73.43%; | H, 9.15%; | N, 2.60%; | S, 7.50%. |
| Found: | C, 72.33%; | H, 9.10%; | N, 2.51%; | S, 8.28%. |

EXAMPLE 6

4-Benzoyloxy-2,2,6,6-tetramethyl-1-(2-acetoxy-3-phenylthiopropyl)piperidine (Compound No. 55)

Following a procedure similar to that of Example 2, 1.34 g (3.13 mmol) of 4-benzoyloxy-2,2,6,6-tetramethyl-1-(2-hydroxy-3-phenylthiopropyl)piperidine, prepared as described in Example 5, was acetylated, and the crude product was purified by silica gel column chromatography (the eluent used was ethyl acetate : hexane in a ratio of 1 : 5 by volume) to afford 0.9 g (yield: 61.2%) of the target compound as a viscous oil.
Infrared Absorption Spectrum (neat) $\nu_{max}$ $cm^{-1}$:
1738, 1715, 1279, 1251, 1114

| Elemental analysis: | | | | |
|---|---|---|---|---|
| Calculated for $C_{27}H_{35}NO_4S$ | | | | |
| | C, 73.43%; | H, 9.15%; | N, 2.60%; | S, 6.83%. |
| Found: | C, 72.33%; | H, 9.10%; | N, 2.51%; | S, 6.98%. |

EXAMPLE 7

4-Benzoyloxy-2,2,6,6(tetramethyl-1-(2-hydroxy-3-methoxypropyl)piperidine (Compound No. 17)

Following a procedure similar to that of Example 1, but using 2.6 g (10 mmol) of 4-benzoyloxy-2,2,6,6-tetramethylpiperidine and 27.0 ml (30 mmol) of glycidyl methyl ether, and purifying the product by silica gel column chromatography (the eluent used was ethyl acetate : hexane in a ratio of 1 : 10 by volume), 3.26 g (yield: 93.3%) of the target compound was obtained as a viscous oil.
Infrared Absorption Spectrum (neat) $\nu_{max}$ $cm^{-1}$:
3400, 1715, 1278, 1113

| Elemental analysis: | | | |
|---|---|---|---|
| Calculated for $C_{20}H_{31}NO_4$ | | | |
| | C, 68.74%; | H, 8.94%; | N, 4.01%. |
| Found: | C, 67.20%; | H, 8.77%; | N, 3.83%. |

EXAMPLE 8

4-Benzoyloxy-2,2,6,6-tetramethyl-1-(2-acetoxy-3-methoxypropyl)piperidine (Compound No. 18)

Following a procedure similar to that of Example 2, 1.5 g (4.29 mmol) of 4-benzoyloxy-2,2,6,6-tetramethyl-1-(2-hydroxy-3-methoxypropyl)piperidine, prepared as described in Example 7, was acetylated, and the crude product was purified over silica gel column chromatography (the eluent used was ethyl acetate : hexane in a ratio of 1 : 10 by volume) to afford 1.37 g (yield: 81.5%) of the target compound as a viscous oil.
Infrared Absorption Spectrum (neat) $\nu_{max}$ $cm^{-1}$:
1737, 1715, 1278, 1243, 1113

| Elemental analysis: | | | |
|---|---|---|---|
| Calculated for $C_{22}H_{33}NO_5$ | | | |
| | C, 67.49%; | H, 8.50%; | N, 3.58%. |
| Found: | C, 63.43%; | H, 8.22%; | N, 3.28%. |

EXAMPLE 9

4-Benzoyloxy-2,2,6,6-tetramethyl-1-(2.3-diacetoxypropyl)piperidine (Compound No. 41)

a) 4-Benzoyloxy-2,2,6,6-tetramethyl-1-(2,3-dihydroxy-propyl)piperidine

Following a procedure similar to that described in Japanese (Kokai) Sho-57-136567, corresponding to EP-A-58434, for the preparation of [4-alkanoyloxy-2,2,6,6-tetramethyl-1-(2,3-dihydroxy-propyl)piperidine], 1.5 g (5.74 mmole) of 4-benzoyloxy-2,2,6,6-tetramethyl-piperidine, 1.9 ml (28.7 mmole) of glycidol and 55 mg (0.29 mmole) of p-toluenesulphonic acid were mixed in 6 ml of 1,2-dimethoxyethane and then heated under reflux for 23 hours. At the end of this time, an aqueous sodium hydrogencarbonate solution was added to the reaction mixture, and the mixture was extracted with ethyl acetate, dried over anhydrous magnesium sulphate and concentrated by evaporation under reduced pressure.

The crude mixture was purified by silica gel column chromatography (the eluent used was ethyl acetate : hexane in a ratio of 1 : 5 by volume) to give 1.13 g (yield: 59%) of the target compound as a viscous oil.
Infrared Absorption Spectrum (KBr) $\nu_{max}$ $cm^{-1}$:
3402, 1714, 1280, 715

| Elemental analysis: | | | |
|---|---|---|---|
| Calculated for $C_{19}H_{28}NO_4$ | | | |
| | C, 64.65%; | H, 8.84%; | N, 3.96%. |
| Found: | C, 63.96%; | H, 8.54%; | N, 3.72%. |

b) 4-Benzoyloxy-2,2,6,6-tetramethyl-1-(2,3-diacetoxypropyl)piperidine

Following a procedure similar to that of Example 2, 3.34 g (10 mmol) of 4-benzoyloxy-2,2,6,6-tetramethyl-1-(2,3-dihydroxypropyl)piperidine, prepared as described in a) above, was acetylated, and the crude product was purified by silica gel column chromatography (the eluent used was ethyl acetate : hexane in a ratio of 1 : 5 by volume) to afford 3.51 g (yield: 83.7%) of the target compound as a viscous oil.
Infrared Absorption Spectrum (KBr) $\nu_{max}$ $cm^{-1}$:
1742, 1716, 1279, 1247, 714

| Elemental analysis: | | | |
|---|---|---|---|
| Calculated for $C_{23}H_{33}NO_6$ | | | |
| | C, 65.85%; | H, 7.93%; | N, 3.34%. |
| Found: | C, 64.67%; | H, 7.83%; | N, 3.25%. |

EXAMPLE 10

4-Benzoyloxy-2,2,6,6-tetramethyl-1-(3-allyloxy-2-hydroxypropyl)piperidine (Compound No. 19)

Following a procedure similar to that of Example 1, but using 4.0 g (15.3 mmol) of 4-benzoyloxy-2,2,6,6-tetramethylpiperidine and 5.4 ml (45.9 mmol) of allyl glycidyl ether, and purifying the product by silica gel column chromatography (the eluent used was ethyl acetate : hexane in a ratio of 1 : 10 by volume), the target compound was obtained as 4.08 g (yield: 71.0%) of a viscous oil.

Infrared Absorption Spectrum (neat) $\nu_{max}$ $cm^{-1}$:

3410, 1715, 1278, 1113

| Elemental analysis: | | | |
|---|---|---|---|
| Calculated for $C_{22}H_{33}NO_4$ | | | |
| | C, 70.37%; | H, 8.86%; | N, 3.73%. |
| Found: | C, 70.18%; | H, 8.75%; | N, 3.62%. |

EXAMPLE 11

4-Benzoyloxy-2,2,6,6-tetramethyl-1-(2-acetoxy-3-allyloxypropyl)piperidine (Compound No. 20)

Following a procedure similar to that of Example 2, 2.96 g (7.88 mmol) of 4-benzoyloxy-2,2,6,6-tetramethyl-1-(3-allyloxy-2-hydroxypropyl)piperidine, prepared as described in Example 10, was acetylated, and the crude product was purified by silica gel column chromatography (the eluent used was ethyl acetate : hexane in a ratio of 1 : 5 by volume) to afford 2.79 g (yield: 84.8%) of the target compound as a viscous oil.

Infrared Absorption Spectrum (neat) $\nu_{max}$ $cm^{-1}$:

1737, 1716, 1278, 1243, 1114

| Elemental analysis: | | | |
|---|---|---|---|
| Calculated for $C_{24}H_{35}NO_5$ | | | |
| | C, 69.04%; | H, 8.45%; | N, 3.35%. |
| Found: | C, 68.84%; | H, 8.28%; | N, 3.22%. |

EXAMPLE 12

4-Benzoyloxy-2,2,6,6-tetramethyl-1-[2-hydroxy-3-(2-methylphenoxy)propyl]piperidine (Compound No. 25)

Following a procedure similar to that of Example 1, but using 5.23 g (20 mmol) of 4-benzoyloxy-2,2,6,6-tetramethylpiperidine and 9.85 g (60 mmol) of glycidyl 2-methylphenyl ether, prepared as described in Preparative Example 2, and purifying the product by silica gel column chromatography (the eluent used was ethyl acetate : hexane in a ratio of 1 : 5 by volume), 5.96 g (yield: 61.4%) of the target compound was obtained as crystals.

Melting point: 70 - 76°C

Infrared Absorption Spectrum (KBr) $\nu_{max}$ $cm^{-1}$:

3420, 1715, 1279, 1116

| Elemental analysis: | | | |
|---|---|---|---|
| Calculated for $C_{26}H_{35}NO_4$ | | | |
| | C, 73.38%; | H, 8.29%; | N, 3.29%. |
| Found: | C, 73.36%; | H, 8.15%; | N, 3.28%. |

EXAMPLE 13

4-Benzoyloxy-2,2,6,6-tetramethyl-1-[2-acetoxy-3-(2-methylphenoxy)propyl]piperidine (Compound No. 26)

Following a procedure similar to that of Example 2, 4.50 g (10.6 mmol) of 4-benzoyloxy-2,2,6,6-tetramethyl-1-[2-hydroxy-3-(2-methylphenoxy)propyl]piperidine, prepared as described in Example 12, was acetylated, and the crude product was purified by silica gel column chromatography (the eluent used was ethyl acetate : hexane in a ratio of 1 : 5 by volume) to afford 4.51 g (yield: 91.2%) of the target compound as a viscous oil.
Infrared Absorption Spectrum (neat) $\nu_{max}$ $cm^{-1}$:
1740, 1715, 1279, 1237, 1115

| Elemental analysis: | | | |
|---|---|---|---|
| Calculated for $C_{28}H_{37}NO_5$ | | | |
| | C, 71.92%; | H, 7.98%; | N, 3.00%. |
| Found: | C, 71.55%; | H, 8.05%; | N, 2.78%. |

EXAMPLE 14

4-Benzoyloxy-2,2,6,6-tetramethyl-1-[2-hydroxy-3-(2,6-dimethylphenoxy)propyl]piperidine (Compound No. 27)

Following a procedure similar to that of Example 1, but using 5.23 g (20 mmol) of 4-benzoyloxy-2,2,6,6-tetramethylpiperidine and 10.7 g (60 mmol) of glycidyl 2,6-dimethylphenyl ether, prepared as described in Preparative Example 3, and purifying the product by silica gel column chromatography (the eluent used was ethyl acetate : hexane in a ratio of 1 : 5 by volume), 5.81 g (yield: 66.0%) of the target compound was obtained as crystals.
Melting point: 104 - 105°C
Infrared Absorption Spectrum (KBr) $\nu_{max}$ $cm^{-1}$:
3200, 1719, 1273, 1116

| Elemental analysis: | | | |
|---|---|---|---|
| Calculated for $C_{27}H_{37}NO_4$ | | | |
| | C, 73.77%; | H, 8.48%; | N, 3.19%. |
| Found: | C, 73.64%; | H, 8.66%; | N, 3.16%. |

EXAMPLE 15

4-Benzoyloxy-2,2,6,6-tetramethyl-1-[2-acetoxy-3-(2,6-dimethylphenoxy)propyl]piperidine (Compound No. 28)

Following a procedure similar to that of Example 2, 2.50 g (5.69 mmol) of 4-benzoyloxy-2,2,6,6-tetramethyl-1-[2-hydroxy-3-(2,6-dimethylphenoxy)propyl]piperidine, prepared as described in Example 14, was acetylated, and the crude product was purified by silica gel column chromatography (the eluent used was ethyl acetate : hexane in a ratio of 1 : 5 by volume) to afford 2.0 g (yield: 74.8%) of the target compound as a viscous oil.
Infrared Absorption Spectrum (neat) $\nu_{max}$ $cm^{-1}$:
1740, 1716, 1278, 1242, 1114

| Elemental analysis: | | | |
|---|---|---|---|
| Calculated for $C_{29}H_{39}NO_5$ | | | |
| | C, 72.32%; | H, 8.16%; | N, 2.91%. |
| Found: | C, 70.79%; | H, 8.09%; | N, 2.72%. |

EXAMPLE 16

4-Benzoyloxy-2,2,6,6-tetramethyl-1-[2-hydroxy-3-(2-t-butylphenoxy)propyl]piperidine (Compound No. 29)

Following a procedure similar to that of Example 1, but using 4.0 g (15.3 mmol) of 4-benzoyloxy-2,2,6,6-tetramethylpiperidine and 9.47 g (45.9 mmol) of glycidyl 2-t-butylphenyl ether, prepared as described in Preparative Example 4, and purifying the product by silica gel column chromatography (the eluent used was ethyl acetate : hexane in a ratio of 1 : 5 by volume), 4.74 g (yield: 61.3%) of the target compound was obtained as crystals.

Melting point: 124 - 126°C

Infrared Absorption Spectrum (KBr) $\nu_{max}$ $cm^{-1}$:

3235, 1717, 1274, 1112

| Elemental analysis: | | | |
|---|---|---|---|
| Calculated for $C_{29}H_{41}NO_4$ | | | |
| | C, 74.48%; | H, 8.84%; | N, 3.00%. |
| Found: | C, 74.68%; | H, 8.98%; | N, 2.90%. |

EXAMPLE 17

4-Benzoyloxy-2,2,6,6-tetramethyl-1-[2-acetoxy-3-(2-t-butylphenoxy)propyl]piperidine (Compound No. 30)

Following a procedure similar to that of Example 2, 3.32 g (7.1 mmol) of 4-benzoyloxy-2,2,6,6-tetramethyl-1-[2-hydroxy-3-(2-t-butylphenoxy)propyl]piperidine, prepared as described in Example 16, was acetylated, and the crude product was purified by silica gel column chromatography (the eluent used was ethyl acetate hexane in a ratio of 1 : 5 by volume) to afford 3.30 g (yield: 91.2%) of the target compound as an amorphous powder.

Infrared Absorption Spectrum (KBr) $\nu_{max}$ $cm^{-1}$:

1741, 1715, 1279, 1227, 1113

| Elemental analysis: | | | |
|---|---|---|---|
| Calculated for $C_{31}H_{43}NO_5$ | | | |
| | C, 73.05%; | H, 8.50%; | N, 2.75%. |
| Found: | C, 72.37%; | H, 8.43%; | N, 2.70%. |

EXAMPLE 18

4-Benzoyloxy-2,2,6,6-tetramethyl-1-[2-hydroxy-3-(4-t-butylphenoxy)propyl]piperidine (Compound No. 31)

Following a procedure similar to that of Example 1, but using 4.0 g (15.3 mmol) of 4-benzoyloxy-2,2,6,6-tetramethylpiperidine and 9.47 g (45.9 mmol) of glycidyl 4-t-butylphenyl ether, prepared as described in Preparative Example 1, and purifying the product by silica gel column chromatography (the eluent used was ethyl

acetate : hexane in a ratio of 1 : 10 by volume), 5.24 g (yield: 73.2%) of the target compound was obtained as crystals.
Melting point: 111 - 112°C
Infrared Absorption Spectrum (KBr) $\nu_{max}$ $cm^{-1}$:
3300, 1715, 1278, 1114

| Elemental analysis: | | | |
|---|---|---|---|
| Calculated for $C_{29}H_{41}NO_4$ | | | |
| | C, 74.48%; | H, 8.84%; | N, 3.00%. |
| Found: | C, 74.44%; | H, 8.78%; | N, 2.86%. |

EXAMPLE 19

4-Benzoyloxy-2,2,6,6-tetramethyl-1-[2-acetoxy-3-(4-t-butylphenoxy)propyl]piperidine (Compound No. 32)

Following a procedure similar to that of Example 2, 4.1 g (8.55 mmol) of 4-benzoyloxy-2,2,6,6-tetramethyl-1-[2-hydroxy-3-(4-t-butylphenoxy)propyl]piperidine, prepared as described in Example 18, was acetylated, and the crude product was purified by silica gel column chromatography (the eluent used was ethyl acetate : hexane in a ratio of 1 : 10 by volume) to afford 3.88 g (yield: 89.0%) of the target compound as a viscous oil.
Infrared Absorption Spectrum (neat) $\nu_{max}$ $cm^{-1}$:
1738, 1716, 1276, 1236, 1114

| Elemental analysis: | | | |
|---|---|---|---|
| Calculated for $C_{31}H_{43}NO_5$ | | | |
| | C, 73.05%; | H, 8.50%; | N, 2.75%. |
| Found: | C, 73.07%; | H, 8.70%; | N, 2.72%. |

EXAMPLE 20

4-Benzoyloxy-2,2,6,6-tetramethyl-1-[2-hydroxy-3-(2-t-butyl-6-methylphenoxy)propyl]piperidine (Compound No. 34)

Following a procedure similar to that of Example 1, but using 4.0 g (15.3 mmol) of 4-benzoyloxy-2,2,6,6-tetramethylpiperidine and 10.1 g (45.9 mmol) of glycidyl 2-t-butyl-6-methylphenyl ether, prepared as described in Preparative Example 5, and purifying the product by silica gel column chromatography (the eluent used was ethyl acetate : hexane in a ratio of 1 : 10 by volume), 1.03 g (yield: 14.0%) of the target compound was obtained as crystals.
Melting point: 104 - 105°C
Infrared Absorption Spectrum (KBr) $\nu_{max}$ $cm^{-1}$:
3280, 1713, 1277, 1114

| Elemental analysis: | | | |
|---|---|---|---|
| Calculated for $C_{30}H_{43}NO_4$ | | | |
| | C, 74.81%; | H, 9.00%; | N, 2.91%. |
| Found: | C, 74.75%; | H, 8.99%; | N, 2.91%. |

EXAMPLE 21

4-Benzoyloxy-2,2,6,6-tetramethyl-1-[2-hydroxy-3-(3,5-di-t-butyl-4-hydroxyphenoxy)propyl]piperidine (Compound No. 36)

Following a procedure similar to that of Example 1, but using 3.14 g (20 mmol) of 4-benzoyloxy-2,2,6,6-tetramethylpiperidine and 10.0 g (35.9 mmol) of glycidyl 3,5-di-t-butyl-4-hydroxyphenyl ether, prepared as described in Preparative Example 6, and purifying the product by silica gel column chromatography (the eluent used was ethyl acetate : hexane in a ratio of 1 : 10 by volume), the target compound was obtained as 3.52 g (yield: 41.8%) of crystals.
Melting point: 186 - 187°C
Infrared Absorption Spectrum (KBr) $\nu_{max}$ $cm^{-1}$:
3575, 1712, 1276, 1059

| Elemental analysis: | | | |
|---|---|---|---|
| Calculated for $C_{33}H_{49}NO_5$ | | | |
| | C, 73.43%; | H, 9.15%; | N, 2.60%. |
| Found: | C, 72.33%; | H, 9.10%; | N, 2.51%. |

EXAMPLE 22

4-Benzoyloxy-2,2,6,6-tetramethyl-1-[2-acetoxy-3-(3,5-di-t-butyl-4-hydroxyphenoxy)propyl]piperidine (Compound No. 37)

Following a procedure similar to that of Example 2, 2.47 g (4.58 mmol) of 4-benzoyloxy-2,2,6,6-tetramethyl-1-[2-hydroxy-3-(3,5-di-t-butyl-4-hydroxyphenoxy)propyl]-piperidine, prepared as described in Example 21, was acetylated, and the crude product was purified by silica gel column chromatography (the eluent used was ethyl acetate : hexane in a ratio of 1 : 10 by volume) to afford 1.30 g (yield: 48.8%) of the target compound as an amorphous powder.
Melting point: 50 - 53°C
Infrared Absorption Spectrum (KBr) $\nu_{max}$ $cm^{-1}$:
3630, 1737, 1716, 1279, 1244, 1114

| Elemental analysis: | | | |
|---|---|---|---|
| Calculated for $C_{35}H_{51}NO_6$ | | | |
| | C, 72.26%; | H, 8.84%; | N, 2.41%. |
| Found: | C, 71.98%; | H, 9.00%; | N, 2.16%. |

EXAMPLE 23

4-Benzoyloxy-2,2,6,6-tetramethyl-1-[2-hydroxy-3-(4-cumylphenoxy)propyl]piperidine (Compound No. 38)

Following a procedure similar to that of Example 1, but using 4.0 g (15.3 mmol) of 4-benzoyloxy-2,2,6,6-tetramethylpiperidine and 12.3 g (35.9 mmol) of glycidyl 4-cumylphenyl ether, prepared as described in Preparative Example 7, and purifying the product by silica gel column chromatography (the eluent used was ethyl acetate : hexane in a ratio of 1 : 10 by volume), 4.80 g (yield: 59.2%) of the target compound was obtained as a viscous oil.
Infrared Absorption Spectrum (neat) $\nu_{max}$ $cm^{-1}$:
3300, 1715, 1278, 1114

| Elemental analysis: | | | |
|---|---|---|---|
| Calculated for $C_{34}H_{43}NO_4$ | | | |
| | C, 77.09%; | H, 8.18%; | N, 2.64%. |
| Found: | C, 77.12%; | H, 8.39%; | N, 2.55%. |

EXAMPLE 24

4-Benzoyloxy-2,2,6,6-tetramethyl-1-[2-acetoxy-3-(4-cumylphenoxy)propyl]piperidine (Compound No. 39)

Following a procedure similar to that of Example 2, 3.68 g (6.95 mmol) of 4-benzoyloxy-2,2,6,6-tetramethyl-1-[2-hydroxy-3-(4-cumylphenoxy)propyl]piperidine, prepared as described in Example 23, was acetylated, and the crude product was purified by silica gel column chromatography (the eluent used was ethyl acetate : hexane in a ratio of 1 : 5 by volume) to afford 3.72 g (yield: 93.7%) of the target compound as a viscous oil.
Infrared Absorption Spectrum (neat) $\nu_{max}$ $cm^{-1}$:
1738, 1715, 1278, 1236, 1114

| Elemental analysis: | | | |
|---|---|---|---|
| Calculated for $C_{36}H_{45}NO_5$ | | | |
| | C, 75.63%; | H, 7.93%; | N, 2.45%. |
| Found: | C, 74.96%; | H, 8.15%; | N, 2.30%. |

EXAMPLE 25

4-Benzoyloxy-2,2,6,6-tetramethyl-1-[2-hydroxy-3-(4-t-butylbenzoyloxy)propyl]piperidine (Compound No. 48)

Following a procedure similar to that of Example 1, but using 4.0 g (15.3 mmol) of 4-benzoyloxy-2,2,6,6-tetramethylpiperidine and 10.8 g (45.9 mmol) of glycidyl 4-t-butylbenzoate and purifying the product by silica gel column chromatography (the eluent used was ethyl acetate : hexane in a ratio of 1 : 10 by volume), 3.02 g (yield: 39.9%) of the target compound was obtained as a viscous oil.
Infrared Absorption Spectrum (neat) $\nu_{max}$ $cm^{-1}$:
3400, 1716, 1277, 1114

| Elemental analysis: | | | |
|---|---|---|---|
| Calculated for $C_{30}H_{41}NO_5$ | | | |
| | C, 72.70%; | H, 8.34%; | N, 2.83%. |
| Found: | C, 71.99%; | H, 8.47%; | N, 2.77%. |

EXAMPLE 26

4-Benzoyloxy-2,2,6,6-tetramethyl-1-[2-acetoxy-3-(4-t-butylbenzoyloxy)propyl]piperidine (Compound No. 49)

Following a procedure similar to that of Example 2, 2.17 g (4.38 mmol) of 4-benzoyloxy-2,2,6,6-tetramethyl-1-[2-hydroxy-3-(4-t-butylbenzoyloxy)propyl]piperidine, prepared as described in Example 25, was acetylated, and the crude product was purified by silica gel column chromatography (the eluent used was ethyl acetate : hexane in a ratio of 1 : 5 by volume) to afford 1.60 g (yield: 68.0%) of the target compound as a vitreous solid.
Infrared Absorption Spectrum (KBr) $\nu_{max}$ $cm^{-1}$:
1742, 1718, 1278, 1235, 1114

| Elemental analysis: | | | |
|---|---|---|---|
| Calculated for $C_{32}H_{43}NO_6$ | | | |
| | C, 71.48%; | H, 8.06%; | N, 2.60%. |
| Found: | C, 70.48%; | H, 7.86%; | N, 2.63%. |

EXAMPLE 27

4-Benzoyloxy-2,2,6,6-tetramethyl-1-{2-hydroxy-3-[3-(3,5-di-t-butyl-4-hydroxyphenyl)propionyloxy]propyl}piperidine (Compound No. 52)

Following a procedure similar to that of Example 1, but using 1.31 g (5.0 mmol) of 4-benzoyloxy-2,2,6,6-tetramethylpiperidine and 5.0 g (15.0 mmol) of glycidyl 3-(3,5-di-t-butyl-4-hydroxyphenyl)propionate, prepared as described in Preparative Example 9, and purifying the product by silica gel column chromatography (the eluent used was ethyl acetate : hexane in a ratio of 1 : 5 by volume), 1.98 g (yield: 67.1%) of the target compound was obtained as a viscous oil.
Infrared Absorption Spectrum (neat) $\nu_{max}$ $cm^{-1}$:
3520, 1717, 1278, 756, 713

| Elemental analysis: | | | |
|---|---|---|---|
| Calculated for $C_{36}H_{53}NO_6$ | | | |
| | C, 72.57%; | H, 8.97%; | N, 2.35%. |
| Found: | C, 71.85%; | H, 8.81%; | N, 2.17%. |

EXAMPLE 28

4-Benzoyloxy-2,2,6,6-tetramethyl-1-{2-hydroxy-3-[3-(3-t-butyl-4-hydroxy-5-methylphenyl)propionyloxy]propyl}piperidine (Compound No. 53)

Following a procedure similar to that of Example 1, but using 0.784 g (3 mmol) of 4-benzoyloxy-2,2,6,6-tetramethylpiperidine and 2.92 g (10 mmol) of glycidyl 3-(3-t-butyl-4-hydroxy-5-methylphenyl)propionate, prepared as described in Preparative Example 10, and purifying the product by silica gel column chromatography (the eluent used was ethyl acetate : hexane in a ratio of 1 : 5 by volume), 1.12 g (yield: 67.5%) of the target compound was obtained as an amorphous powder.
Infrared Absorption Spectrum (KBr) $\nu_{max}$ $cm^{-1}$:
3488, 1716, 1279, 1175, 713

| Elemental analysis: | | | |
|---|---|---|---|
| Calculated for $C_{33}H_{47}NO_6$ | | | |
| | C, 71.58%; | H, 8.56%; | N, 2.53%. |
| Found: | C, 70.29%; | H, 8.44%; | N, 2.13%. |

EXAMPLE 29

Di[2,2,6,6-tetramethyl-1-(2-hydroxy-3-phenoxypropyl)-4-piperidyl] succinate (Compound No. 74)

Following a procedure similar to that of Example 1, but using 3.0 g (7.56 mmol) of di(2,2,6,6-tetramethyl-4-piperidyl) succinate and 6.23 g (30.2 mmol) of glycidyl phenyl ether, and purifying the product by silica gel

column chromatography (the eluent used was ethyl acetate : hexane in a ratio of 1 : 5 by volume), 4.60 g (yield: 83.6%) of the target compound were obtained as an amorphous powder.
Infrared Absorption Spectrum (KBr) $\nu_{max}$ $cm^{-1}$:
3231, 1727, 1243, 1134

| Elemental analysis: | | | |
|---|---|---|---|
| Calculated for $C_{40}H_{60}N_2O_8$ | | | |
| | C, 68.94%; | H, 8.68%; | N, 4.02%. |
| Found: | C, 68.22%; | H, 8.68%; | N, 3.96%. |

EXAMPLE 30

Di{2,2,6,6-tetramethyl-1-[2-hydroxy-3-(4-t-butylphenoxy)propyl]-4-piperidyl} succinate (Compound No. 78)

Following a procedure similar to that of Example 1, but using 3.0 g (7.56 mmol) of di(2,2,6,6-tetramethyl-4-piperidyl) succinate and 6.23 g (30.2 mmol) of glycidyl 4-t-butylphenyl ether, prepared as described in Preparative Example 1, and purifying the product by silica gel column chromatography (the eluent used was ethyl acetate : hexane in a ratio of 1 : 5 by volume), 4.60 g (yield: 75.2%) of the target compound was obtained as an amorphous powder.
Infrared Absorption Spectrum (KBr) $\nu_{max}$ $cm^{-1}$:
3446, 1733, 1249, 1182

| Elemental analysis: | | | |
|---|---|---|---|
| Calculated for $C_{48}H_{76}N_2O_8$ | | | |
| | C, 71.25%; | H, 9.47%; | N, 3.46%. |
| Found: | C, 71.19%; | H, 9.56%; | N, 3.46%. |

EXAMPLE 31

Di[2,2,6,6-tetramethyl-1-(2-hydroxy-3-phenoxypropyl)-4-piperidyl] adipate (Compound No. 86)

Following a procedure similar to that of Example 1, but using 3.0 g (7.07 mmol) of di(2,2,6,6-tetramethyl-4-piperidyl) adipate and 5.74 ml (42.4 mmol) of glycidyl phenyl ether, and purifying the product by silica gel column chromatography (the eluent used was ethyl acetate : hexane in a ratio of 1 : 5 by volume), 4.14 g (yield: 80.8%) of the target compound was obtained as crystals.
Melting point: 136 - 137°C
Infrared Absorption Spectrum (KBr) $\nu_{max}$ $cm^{-1}$:
3220, 1727, 1225, 1142

| Elemental analysis: | | | |
|---|---|---|---|
| Calculated for $C_{42}H_{64}N_2O_8$ | | | |
| | C, 69.58%; | H, 8.90%; | N, 3.86%. |
| Found: | C, 69.47%; | H, 8.88%; | N, 3.91%. |

EXAMPLE 32

Di{2,2,6,6-tetramethyl-1-[2-hydroxy-3-(4-t-butylphenoxy)propyl]-4-piperidyl} adipate (Compound No. 90)

Following a procedure similar to that of Example 1, but using 3.0 g (7.07 mmol) of di(2,2,6,6-tetramethyl-4-piperidyl) adipate and 5.84 g (28.3 mmol) of glycidyl 4-t-butylphenyl ether, prepared as described in Preparative Example 1, and purifying the product by silica gel column chromatography (the eluent used was ethyl acetate : hexane in a ratio of 1 : 10 by volume), 3.70 g (yield: 62.6%) of the target compound was obtained as an amorphous powder.
Infrared Absorption Spectrum (KBr) $\nu_{max}$ $cm^{-1}$:
3450, 1732, 1248, 1181

| | | | |
|---|---|---|---|
| Elemental analysis: | | | |
| Calculated for $C_{50}H_{80}N_2O_8$ | | | |
| | C, 71.73%; | H, 9.63%; | N, 3.35%. |
| Found: | C, 71.41%; | H, 9.44%; | N, 3.32%. |

EXAMPLE 33

Di{2,2,6,6-tetramethyl-1-[2-hydroxy-3-(2-t-butylphenoxy)propyl]-4-piperidyl} sebacate (Compound No. 142)

Following a procedure similar to that of Example 1, but using 4.33 g (9.0 mmol) of di(2,2,6,6-tetramethyl-4-piperidyl) sebacate and 11.1 g (54 mmol) of glycidyl 2-t-butylphenyl ether, prepared as described in Preparative Example 4, and purifying the product by silica gel column chromatography (the eluent used was ethyl acetate : hexane in a ratio of 1 : 5 by volume), 2.27 g (yield: 28.2%) of the target compound was obtained as a vitreous solid.
Infrared Absorption Spectrum (neat) $\nu_{max}$ $cm^{-1}$:
3430, 1730, 1232, 1175

| | | | |
|---|---|---|---|
| Elemental analysis: | | | |
| Calculated for $C_{54}H_{88}N_2O_8$ | | | |
| | C, 72.61%; | H, 9.93%; | N, 3.14%. |
| Found: | C, 71.05%; | H, 9.54%; | N, 2.91%. |

EXAMPLE 34

Di{2,2,6,6-tetramethyl-1-[2-hydroxy-3-(4-t-butylphenoxy)propyl]-4-piperidyl} sebacate (Compound No. 144)

Following a procedure similar to that of Example 1, but using 4.8 g (10 mmol) of di(2,2,6,6-tetramethyl-4-piperidyl) sebacate and 12.4 g (60 mmol) of glycidyl 4-t-butylphenyl ether, prepared as described in Preparative Example 1, and purifying the product by silica gel column chromatography (the eluent used was ethyl acetate : hexane in a ratio of 1 : 5 by volume), 6.95 g (yield: 77.9%) of the target compound was obtained as crystals.
Melting point: 107 - 108°C
Infrared Absorption Spectrum (KBr) $\nu_{max}$ $cm^{-1}$:
3225, 1732, 1251, 1162

| Elemental analysis: | | | |
|---|---|---|---|
| Calculated for $C_{54}H_{88}N_2O_8$ | | | |
| | C, 72.61%; | H, 9.93%; | N, 3.14%. |
| Found: | C, 72.54%; | H, 10.00%; | N, 3.09%. |

EXAMPLE 35

Di{2,2,6,6-tetramethyl-1-[2-acetoxy-3-(4-t-butylphenoxy)propyl]-4-piperidyl} sebacate Compound No. 145)

Following a procedure similar to that of Example 2, 3.5 g (3.92 mmol) of di(2,2,6,6-tetramethyl-1-[2-hydroxy-3-(4-t-butylphenoxy)propyl]-4-piperidyl} sebacate, prepared as described in Example 34, was acetylated, and the crude product was purified by silica gel column chromatography (the eluent used was ethyl acetate : hexane in a ratio of 1 : 10 by volume) to afford 2.52 g (yield: 64.5%) of the target compound as a viscous oil.
Infrared Absorption Spectrum (neat) $\nu_{max}$ $cm^{-1}$:
1735, 1236, 1182

| Elemental analysis: | | | |
|---|---|---|---|
| Calculated for $C_{58}H_{92}N_2O_{10}$ | | | |
| | C, 71.28%; | H, 9.49%; | N, 2.87%. |
| Found: | C, 70.52%; | H, 9.58%; | N, 2.68%. |

PREPARATIVE EXAMPLE 1

Glycidyl 4-t-butylphenyl ether

A suspension was obtained by adding 50 ml of anhydrous dimethyl sulphoxide to 3.23 g (73.3 mmol) of sodium hydride (as a 55% w/w suspension in mineral oil) which had been washed with dry hexane under a nitrogen atmosphere. 10.0 g (66.6 mmol) of 4-t-butylphenol was added slowly, dropwise, to the suspension, with ice cooling. After the 4-t-butylphenol had been added, the mixture was allowed to return to room temperature and was then stirred for one hour. 15.5 ml (200 mmol) of epichlorhydrin was added to the mixture, which was then stirred at 40°C for one hour. At the end of this time, 300 ml of water was added to the reaction mixture, which was then extracted three times, each time with 100 ml of ethyl acetate. The organic layers were combined, brought to neutrality with dilute aqueous hydrochloric acid, washed with a saturated aqueous sodium chloride solution, dried over anhydrous sodium sulphate and concentrated by evaporation under reduced pressure to afford an oil. The oil was purified by distillation under reduced pressure (140 150/2-2.5 mmHg) to obtain 13.3 g (yield: 96.9%) of glycidyl 4-t-butylphenyl ether.
Nuclear Magnetic Resonance Spectrum ($CDCl_3$) $\delta$ ppm:
7.30 (doublet, J=8Hz, 2H),
6.85 (doublet, J=8Hz, 2H),
4.30-4.07 (doublet of doublets, J=3 & 10Hz, 1H),
4.03-3.78 (doublet of doublets, J=5 & 10Hz, 1H),
3.45-3.15 (multiplet, 1H),
2.95-2.60 (multiplet, 2H),
1.35 (singlet, 9H).

Following similar procedures to that of Preparative Example 1, but using appropriate starting materials, quantities and conditions, the compounds of Preparative Examples 2 to 7 were prepared.

PREPARATIVE EXAMPLE 2

Glycidyl 2-methylphenyl ether

The target compound was obtained as an oil.
Nuclear Magnetic Resonance Spectrum (90 MHz, $CDCl_3$) δ ppm:
6.70-7.35 (multiplet, 4H),
4.23 (doublet of doublets, J=4.5 & 17Hz, 1H),
3.96 (doublet of doublets, J=7.5 & 17Hz, 1H),
3.20-3.46 (multiplet, 1H),
2.66-2.95 (multiplet, 2H),
2.24 (singlet, 3H).

PREPARATIVE EXAMPLE 3

Glycidyl 2,6-dimethylphenyl ether

The target compound was obtained as an oil.
Nuclear Magnetic Resonance Spectrum (90 MHz, $CDCl_3$) δ ppm:
6.80-7.20 (multiplet, 3H),
4.04 (doublet of doublets, J=4.5 & 16Hz, 1H),
3.75 (doublet of doublets, J=7.5 & 16Hz, 1H),
3.20-3.50 (multiplet, 1H),
2.60-2.96 (multiplet, 2H),
2.28 (singlet, 6H).

PREPARATIVE EXAMPLE 4

Glycidyl 2-t-butylphenyl ether

The target compound was obtained as an oil.
Nuclear Magnetic Resonance Spectrum (90 MHz, $CDCl_3$) δ ppm:
6.71-7.43 (multiplet, 4H),
4.24 (doublet of doublets, J=5 & 16Hz, 1H),
3.96 (doublet of doublets, J=7 & 16Hz, 1H),
3.23-3.55 (multiplet, 1H),
2.65-3.00 (multiplet, 2H),
1.42 (singlet, 9H).

PREPARATIVE EXAMPLE 5

Glycidyl 2-t-butyl-6-methylphenyl ether

The target compound was obtained as an oil.
Nuclear Magnetic Resonance Spectrum (90 MHz, $CDCl_3$) δ ppm:
6.80-7.30 (multiplet, 3H),
3.95-4.25 (doublet of doublets, J=4.5 & 16Hz, 1H),
3.75-3.95 (doublet of doublets, J=7.5 & 16.5Hz, 1H),
3.20-3.55 (multiplet, 1H),
2.75-3.00 (multiplet, 2H),
2.30 (singlet, 3H),
1.42 (singlet, 9H).

PREPARATIVE EXAMPLE 6

Glycidyl 3,5-di-t-butyl-4-hydroxyphenyl ether

The target compound was obtained as crystals having a melting point of 43 - 43.7°C.

Nuclear Magnetic Resonance Spectrum (90 MHz, $CDCl_3$) δ ppm:
6.84 (singlet, 2H),
4.80 (singlet, 1H),
3.80-4.30 (multiplet, 2H),
3.20-3.46 (multiplet, 1H),
2.65-2.97 (multiplet, 2H),
1.44 (singlet, 18H).

PREPARATIVE EXAMPLE 7

Glycidyl 4-cumylphenyl ether

The target compound was obtained as an oil.
Nuclear Magnetic Resonance Spectrum (90 MHz, $CDCl_3$) δ ppm:
7.30-7.15 (multiplet, 5H),
7.15 (doublet, 8.6Hz, 2H),
6.80 (doublet, 8.6Hz, 2H),
4.14-4.19 (doublet of doublets, J=3.3 & 11.2Hz, 1H),
3.90-3.95 (doublet of doublets, J=5.9 & 11.2Hz, 1H),
3.40-3.30 (singlet, 1H),
2.90 (triplet, J=4.0Hz, 1H),
2.70-2.80 (multiplet, 1H),
0.65 (singlet, 6H).

PREPARATIVE EXAMPLE 8

Glycidyl Phenyl Thioether

150 ml of toluene, 33.1 g (0.3 mol) of thiophenol, 35 ml of triethylamine and 32.0 g (0.35 mol) of epichlorhydrin were combined to form a solution which was stirred at room temperature for 1 hour. 300 ml of water was added to the stirred solution, and the resulting mixture was shaken well. The toluene layer was separated and washed with a saturated aqueous solution of sodium chloride, dried over anhydrous sodium sulphate, and then concentrated by evaporation under reduced pressure to afford an oil. The resulting oil was then mixed with a solution of 20 g potassium hydroxide in 150 ml methanol and the mixture was left to stand for 1 hour at room temperature. The solution which was obtained was then condensed by evaporation under reduced pressure and the residue was dissolved in 200 ml of ethyl acetate. The ethyl acetate layer was washed with water, dried over anhydrous sodium sulphate, and concentrated by evaporation under reduced pressure to afford an oil. The oil thus obtained was purified by silica gel column chromatography (the eluent used was hexane followed by ethyl acetate : hexane in a ratio of 1 : 3 by volume) to isolate the target compound which was then concentrated by evaporation under reduced pressure to afford an oil. The oil was purified by distillation under reduced pressure (140 150/2-2.5 mmHg) to obtain 25.8 g (yield 52.0%) of glycidyl phenyl thioether.
Nuclear Magnetic Resonance Spectrum ($CDCl_3$) δ ppm:
7.50-7.20 (multiplet, 5H),
3.30-2.65 (multiplet, 4H),
2.50 (multiplet, 1H).

The compounds of Preparative Examples 9 and 10 were prepared according to the method described in J. Org.Chem., (1961), 26, 2681.

PREPARATIVE EXAMPLE 9

Glycidyl-3-(3,5-di-t-butyl-4-hydroxyphenyl)propionate

The target compound was prepared as an oil having a boiling point of 178 - 185°C at a pressure of 1 mmHg.
Nuclear Magnetic Resonance Spectrum (90 MHz, $CDCl_3$) δ ppm:
6.97 (singlet, 1H),
4.66 (singlet, 1H),
4.40 (doublet of doublets, J=3 & 12Hz, 1H),
3.93 (doublet of doublets, J=6 & 12Hz, 1H),

3.07-3.30 (multiplet, 6H),
2.50-3.00 (multiplet, 6H),
1.40 (singlet, 18H).

PREPARATIVE EXAMPLE 10

Glycidyl-3-(3-t-butyl-4-hydroxy-5-methylphenyl)propionate

The target compound was prepared as crystals having a melting point of 45 - 46°C.

Nuclear Magnetic Resonance Spectrum (90 MHz, $CDCl_3$) δ ppm:
6.97 (doublet, J=2Hz, 1H),
6.83 (doublet, J=2Hz, 1H),
4.66 (singlet, 1H),
4.40 (doublet of doublets, J=3 & 12Hz, 1H),
3.93 (doublet of doublets, J=6 & 12Hz, 1H),
3.07-3.30 (multiplet, 6H),
2.50-3.00 (multiplet, 6H),
2.20 (singlet, 3H),
1.40 (singlet, 9H).

EXAMPLE 36

Photostability Test

The photostability of polymeric materials containing various photostabilisers was assayed as follows.

100 parts of an unstabilised polypropylene powder (melt flow rate of < 11), 0.2 part of stearyl 3-(3,5-di-t-butyl-4-hydroxyphenyl)propionate (Irganox™ 1076) and 0.25 part of photostabiliser were kneaded in a mixer (Laboplasto™ mill, manufactured by Toyo Seiki Seisakusho) at 200°C for 10 minutes, to homogeneity. The resulting homogeneous preparation was rolled into a sheet of about 2 - 3 mm thickness, using a water-cooled hydraulic press.

A part of the resulting sheet was hydraulically pressed at 260°C for 6 minutes and then dipped immediately into cold water to obtain a sheet of 0.5 mm thickness. Following a similar procedure, a 0.1 mm thick film was formed from the 0.5 mm thick sheet. The thus obtained film was cut into test pieces measuring 50 x 120 mm.

The test pieces were exposed to light in a carbon arc sunshine weather meter at a black panel temperature of 63 ± 3°C, and were periodically tested for tensile strength. The half-life ($T_{1/2}$) was measured for the test pieces, the half-life being the time at which elongation has dropped to 50% of the value at $T_0$ (starting time). The ratios of the $T_{1/2}$ values for the stabilisers of the present invention to that of a control containing no stabiliser were determined in order to provide an Index of Efficacy (I.F.). Results are shown in Table 2, following Example 39.

EXAMPLE 37

Heat-Stability Test

A portion of the 0.5 mm thick polypropylene sheet prepared in Example 36 was used to obtain test pieces measuring 10 x 100 mm. These test pieces were heated in a 150°C oven until they became brittle. Brittleness was determined by bending the pieces through 180°, and the amount of time (in days) until the test pieces became brittle was recorded, to provide an Index of Efficacy. Results are shown in Table 2, following Example 39.

EXAMPLE 38

Latex Migration Test

A 0.1 mm layer of a latex was applied, using an applicator, to each surface of a 50 x 120 mm sheet of a 0.1 mm thickness film prepared as described in Example 36. The latex was an emulsion consisting of Primal™ HA-8 (50%) and Primal ASE-60 (5%) (both manufactured by Nippon Acryl Kagaku K.K.), aqueous ammonia (28% v/v, 1.5%) and water (43.5%). The treated film was then kept at 60°C for 7 days, after which the latex

layers were removed from the film by peeling off. The light resistance of the film was then assayed by the procedure described in Example 36 to obtain $T_{1/2}$. The ratio of the $T_{1/2}$ of test samples to that of control samples containing no such stabiliser was calculated in order to determine the Index of Efficacy (latex), or L.I.F., which provides a measure of the tendency of the stabiliser to migrate into latex. The greater the value of the index, the higher the efficacy of the stabiliser. Results are shown in Table 2, following Example 39.

EXAMPLE 39

Volatility test (TGA)

If photostabilisers volatilise (evaporate, sublimate) by the effect of heat during the production of polymer, then clogging can occur in a vent-type extruder. Accordingly, the volatility of the compounds of the present invention was measured by direct heating.

About 10 mg of sample was introduced into an aluminum container and heated from ambient temperature to 320°C at a rate of 5°C/min. During heating, air was passed through the container at a flow rate of 50 ml/min. The residue (recorded as % total) of crystals in the aluminum container was measured during heating using a thermogravimeter (Model Dupont 2000 System) to provide an index of volatility. Volatility at 200 and 300°C is shown in the following Table, and the results demonstrate that the compounds of the present invention are less volatile than the comparative compounds.

Test results obtained in Examples 36 to 39 are as shown in Table 2. "Light" is used to indicate the tests for photostability, while "Heat" is used to indicate the test for brittleness caused by heating.

Table 2

| Example Cmpd. No. | $T_{1/2}$ | Light IF | LIF | Heat Day | Volatility (%) 200°C / 300°C |
|---|---|---|---|---|---|
| 1 | 670 | 3.7 | 1.4 | 4 | 100.0 / 91.8 |
| 2 | 620 | 3.4 | 2.2 | 4 | 100.0 / 91.6 |
| 3 | 1000 | 5.6 | 5.1 | 7 | 100.0 / 100.0 |
| 4 | 120 | 0.7 | 0.9 | 4 | 100.0 / 91.4 |
| 5 | 120 | 0.7 | 0.8 | 4 | 99.2 / 93.4 |
| 6 | 650 | 3.7 | 1.4 | 4 | 99.1 / 54.9 |
| 7 | 710 | 3.9 | 1.9 | 4 | 97.9 / 56.8 |
| 8 | 760 | 4.2 | 1.8 | 4 | 100.0 / 72.2 |
| 9 | 720 | 4.0 | 1.6 | 4 | 100.0 / 82.6 |
| 10 | 700 | 3.9 | 1.6 | 3 | 100.0 / 55.0 |
| 11 | 570 | 3.2 | 1.6 | 4 | 100.0 / 92.3 |
| 12 | 630 | 3.5 | 1.9 | 4 | 100.0 / 92.4 |
| 13 | 720 | 4.0 | 1.9 | 5 | 100.0 / 99.2 |
| 14 | 630 | 3.5 | 1.5 | 4 | 100.0 / 97.7 |
| 15 | 660 | 3.7 | 2.4 | 5 | 100.0 / 97.5 |
| 16 | 580 | 3.2 | 2.7 | 11 | 98.4 / 90.3 |
| 17 | 610 | 3.4 | 1.8 | 7 | 99.7 / 94.4 |
| 18 | 690 | 3.8 | 1.6 | 4 | 99.9 / 92.9 |
| 19 | 670 | 3.7 | 2.5 | 4 | 100.0 / 93.7 |
| 20 | 790 | 4.4 | 2.7 | 6 | 100.0 / 98.1 |
| 21 | 730 | 4.1 | 2.3 | 4 | 99.7 / 96.5 |
| 22 | 570 | 3.2 | 2.4 | 10 | 99.8 / 94.0 |
| 23 | 710 | 3.9 | 2.0 | 5 | 97.9 / 93.2 |

Table 2 (cont'd)

| Example Cmpd. No. | $T_{1/2}$ | Light IF | LIF | Heat Day | Volatility (%) 200°C / 300°C |
|---|---|---|---|---|---|
| 24 | 590 | 3.3 | 1.9 | 5 | 98.9 / 95.3 |
| 25 | 640 | 3.6 | 2.9 | 7 | 100.0 / 99.4 |
| 26 | 570 | 3.2 | 1.8 | 16 | 99.4 / 95.4 |
| 27 | 460 | 2.6 | 2.7 | 15 | 97.7 / 89.3 |
| 28 | 1090 | 6.1 | 5.0 | 7 | 100.0 / 99.7 |
| 29 | 1010 | 5.7 | 5.9 | 7 | 100.0 / 98.1 |
| 30 | 1060 | 5.9 | 5.1 | 10 | 100.0 / 99.6 |
| 31 | 1060 | 5.9 | 5.1 | 7 | 100.0 / 100.0 |
| 32 | 1020 | 5.7 | 5.1 | 8 | 99.4 / 97.4 |
| 33 | 860 | 5.5 | 5.0 | 11 | 100.0 / 98.8 |
| 34 | 1060 | 5.9 | 5.5 | 10 | 100.0 / 100.0 |
| 35 | 960 | 5.1 | 4.8 | 7 | 99.3 / 51.2 |
| Comparison Compound 1 | 570 | 3.2 | 1.2 | 4 | 73.3 / 3.2 |
| Comparison Compound 2 | 1020 | 5.7 | 2.9 | 5 | 98.3 / 39.0 |
| Control | 180 | 1.0 | 1.0 | 4 | - - |

Comparison compound 1 : 4-Benzoyloxy-2,2,6,6-tetramethylpyridine

Comparison compound 2 : Bis(2,2,6,6-tetramethylpyridyl)-sebacate

EXAMPLE 40

Evaluation In Polyurethane

100 parts of a thermoplastic polyurethane (Paraprane Pellet 22S, trade mark, manufactured by Nippon Polyurethane Kogyo K.K.), 1.0 part of test compound and 0.5 part of tetrakis[3-(3,5-di-t-butyl-4-hydroxyphenyl)-propionyloxymethyl]methane (Irganox™ 1010) were dissolved homogeneously in 400 parts of dimethylformamide. The resulting solution was cast onto a glass plate to a thickness of about 0.4 mm, dried at 70°C

for 30 minutes, then dried at 140°C for 5 minutes, to provide a film having a thickness of about 0.1 mm.

A first part of the film was subjected to light irradiation in a xenon weatherometer at a black panel temperature of 63 ± 3°C with no water spraying for 1200 hours. The yellow index ($YI_{1200}$) of the film was determined after this time according to JIS K7103 (Yellowness of Plastics and Method of Testing Yellowness).

A second part of the (unirradiated) film obtained above was partly exposed to nitrogen oxide gas ($NO_x$), at a level of 1,000 ppm, according to JIS L0855 (Method of Testing Colour Fastness to Nitrogen Oxide Gas) for 16 hours. The yellow index ($YI_{16}$) of the film after this time was determined according to the procedure specified by JIS K7103 (supra). The yellow index before either treatment is designated as $YI_0$. The test results are as shown in Table 3.

Table 3

| Stabiliser (parts) Compound of Example No. | Other | $YI_0$ | $YI_{16}$ | $YI_{1200}$ |
|---|---|---|---|---|
| 3 (1) | - | 1.4 | 4.8 | 40.3 |
| 34 (1) | - | 1.2 | 4.4 | 39.4 |
| 3 (0.5) | Comp. Cpd. 4 (0.5) | 1.7 | 7.2 | 28.4 |
| 34 (0.5) | Comp. Cpd. 4 (0.5) | 1.5 | 7.0 | 31.2 |
| Comp. Cpd. 1 (1) | | 1.6 | 21.1 | 38.5 |
| Comp. Cpd. 3 (1) | | 1.2 | 5.9 | 38.6 |
| Comp. Cpd. 4 (1) | | 1.6 | 14.6 | 39.9 |
| Comp. Cpd. 1 (0.5) | Comp. Cpd. 4 (0.5) | 1.7 | 15.3 | 27.2 |
| Comp. Cpd. 3 (0.5) | Comp. Cpd. 4 (0.5) | 1.4 | 6.0 | 27.2 |
| Blank | | 1.4 | 14.1 | 57.9 |

Comparative compound 1 : 4-Benzoyloxy-2,2,6,6-tetramethylpyridine

Comparative compound 3 : Bis(1,2,2,6,6-pentamethylpyridyl)sebacate

Comparative compound 4 : Tinuvin-P (registered trade mark, Ciba-Geigy AG)

EXAMPLE 41

Evaluation of Heat Resistance in Polyoxymethylene Polymer

0.5 Part of a stabiliser was added to 100 parts of a polyoxymethylene polymer (Duracon™ M 90, manufactured by Polyplastics K.K.) and the resulting mixture was blended in a V-type mixer for 30 minutes. The mixture was then pelletised using an extruder (at a cylinder-1 temperature of 200°C, a cylinder-2 temperature of 210°C and a tie temperature of 210°C). A 2 mm thick sheet measuring 70 mm x 50 mm was prepared from the resulting pellets by injection moulding, and the sheet kept in a 155°C oven until cracking occurred. The heat resistance of the polymer was evaluated according to the number of days taken until cracking occurred.

The yellow indices of the stabilisers were determined prior to mixing with the polymer in accordance with JIS K7103. The test results are shown in Table 4.

Table 4

| Stabiliser | Yellow index $YI_0$ | Cracking (day) |
|---|---|---|
| Example compound 3 | 1.5 | 15 |
| Example compound 34 | 1.8 | 15 |
| Comparative compound 2 | 1.6 | 9 |
| Comparative compound 4 | 6.9 | 14 |
| Comparative compound 5 | 2.1 | 10 |
| Blank | 1.6 | 13 |

Comparative compound 2 : Bis(2,2,6,6-tetramethyl-pyridyl) sebacate
Comparative compound 4 : Tinuvin-P
Comparative compound 5 : Tinuvin-622 (registered trade mark, Ciba-Geigy AG)

**Claims**

**1.** A compound of formula (I):

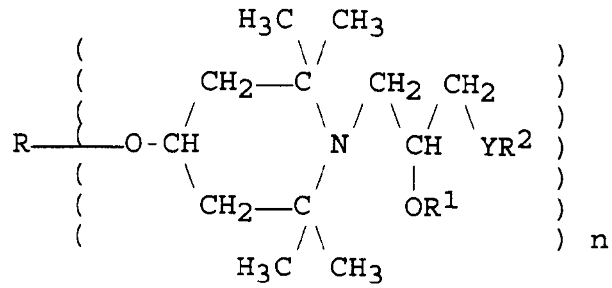

(I)

in which:

R represents a hydrogen atom, a monocarboxylic acyl group or a polycarboxylic acyl group, R having a valency of from 1 to 4;

$R^1$ represents a hydrogen atom or a carboxylic ester moiety,

$R^2$ represents:

an alkyl group,
an alkenyl group,
an alkynyl group,
an aryl group,
an aralkyl group,
a heterocyclic group, or
a heterocyclylalkyl group,

said aryl, aralkyl, heterocyclic and heterocyclylalkyl groups optionally having one or more substituents,

or $R^2$ represents a carboxylic ester moiety;

n is an integer between 1 and 4, inclusive, and is equal to the valency of R; and

Y represents an oxygen atom or a sulphur atom,

**PROVIDED THAT,** when n is 2, 3 or 4, then each $R^1$ and each $R^2$ may be the same or different,

and acid addition salts thereof.

**2.** A compound according to Claim 1, wherein $R^2$ represents:

an alkyl group having from 1 to 18 carbon atoms,
an alkenyl group having from 2 to 6 carbon atoms,
an alkynyl group having from 2 to 6 carbon atoms,
an aryl group having from 6 to 10 carbon atoms, or
an aralkyl group having from 7 to 12 carbon atoms,

said groups being substituted or unsubstituted.

**3.** A compound according to Claim 2, wherein $R^2$ represents:

an alkenyl group having from 3 to 6 carbon atoms, or
an alkynyl group having from 3 to 6 carbon atoms.

**4.** A compound according to any preceding Claim, wherein said substituents are selected from substituents A:

alkyl groups having from 1 to 4 carbon atoms,
cycloalkyl groups having from 3 to 6 carbon atoms,
haloalkyl groups having 1 or 2 carbon atoms,
halogen atoms,
aralkyl groups having from 7 to 12 carbon atoms,
hydroxy groups,
alkoxy groups having from 1 to 4 carbon atoms,
nitro groups,
cyano groups,
carbamoyl groups,
carboxy groups, and
amino groups which are unsubstituted or substituted by at least one alkyl group having from 1 to 4 carbon atoms.

5. A compound according to Claim 4, wherein said substituents A are selected from
    alkyl groups having from 1 to 4 carbon atoms,
    cycloalkyl groups having from 3 to 6 carbon atoms,
    haloalkyl groups having 1 or 2 carbon atoms,
    halogen atoms,
    aralkyl groups having from 7 to 12 carbon atoms and
    hydroxyl groups.

6. A compound according to any preceding Claim, as appropriate, wherein at least one of $R^1$ and $R^2$ represents an ester moiety selected from:
    alkanoyl groups having from 1 to 18 carbon atoms,
    haloalkanoyl groups having from 2 to 4 carbon atoms,
    arylacyl groups having from 7 to 11 carbon atoms, and
    arylalkanoyl groups having from 8 to 13 carbon atoms,
   said groups being unsubstituted or substituted by at least one substituent individually selected from said substituents A defined in Claim 4.

7. A compound according to Claim 1, wherein at least one of $R^1$ and $R^2$ represents an ester moiety selected from:
    alkanoyl groups having from 1 to 18 carbon atoms,
    haloalkanoyl groups having from 2 to 4 carbon atoms,
    arylacyl groups having from 7 to 11 carbon atoms, and
    arylalkanoyl groups having from 8 to 13 carbon atoms.

8. A compound according to any preceding Claim, wherein, when $R^1$ and/or $R^2$ represents a substituted group, then at least one of $R^1$ and $R^2$ is substituted by from one to five substituents individually selected from substituents A defined in Claim 4, provided that $R^1$ cannot represent a substituted hydrogen atom.

9. A compound according to Claim 8, wherein at least one of $R^1$ and $R^2$ is substituted by from one to three substituents individually selected from substituents A.

10. A compound according to any preceding Claim, wherein R represents an alkanedioyl group having from 4 to 12 carbon atoms, an alkanoyl group having from 2 to 4 carbon atoms, or a benzoyl group which is unsubstituted or which is substituted with 1 or 2 alkyl groups having from 1 to 4 carbon atoms.

11. A compound according to any preceding Claim, as appropriate, wherein $R^1$ represents a hydrogen atom or an alkanoyl group having from 2 to 6 carbon atoms, a haloformyl group or a benzoyl group which is unsubstituted or which is substituted with 1 or 2 alkyl groups having from 1 to 4 carbon atoms.

12. A compound according to any preceding Claim, as appropriate, wherein $R^2$ represents a phenyl group which is unsubstituted or which has 1 to 3 substituents selected from
    an alkyl group having from 1 to 4 carbon atoms, an
    aralkyl group having from 7 to 9 carbon atoms, a
    halomethyl group and a hydroxyl group,
   a benzoyl group which is unsubstituted or which is substituted with 1 to 3 alkyl groups each having from 1 to 4 carbon atoms, or an arylalkanoyl group having 9 carbon atoms and which is unsubstituted or which has 1 to 3 substituents selected from alkyl groups having from 1 to 4 carbon atoms and hydroxyl groups.

13. A compound according to any preceding Claim, wherein Y represents an oxygen atom.

14. A compound according to any preceding Claim, wherein R represents an alkanedioyl group having from 8 to 10 carbon atoms.

15. A compound according to any of Claims 1 to 14, as appropriate, wherein R represents a benzoyl group.

16. A compound according to any preceding Claim, as appropriate, wherein $R^1$ represents a hydrogen atom or an acetyl group.

17. A compound according to any preceding Claim, as appropriate, wherein $R^2$ represents a phenyl group which is unsubstituted or which has 1 to 3 substituents selected from alkyl groups having from 1 to 4 carbon

atoms and aralkyl groups having from 7 to 9 carbon atoms.

**18.** A compound according to any preceding Claim, wherein n is 1.

**19.** A compound according to Claim 1, wherein n is 2.

**20.** Di-[2,2,6,6-tetramethyl-1-(2-hydroxy-3-phenoxypropyl)-4-piperidyl] sebacate.

**21.** Di-{2,2,6,6-tetramethyl-1-[2-hydroxy-3-(4-t-butylphenoxy)propyl]-4-piperidyl} sebacate.

**22.** A photostabiliser for polymeric materials having a compound according to any preceding Claim as an active ingredient.

**23.** A polymeric material comprising a compound according to any of Claims 1 to 21 as a stabiliser.

**24.** An article at least partially constructed from a material according to Claim 23.

European Patent Office

# EUROPEAN SEARCH REPORT

Application Number

EP 93 30 6471

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 119 961 (CIBA-GEIGY) 26 September 1984<br>*see BHETP, page 27* | 1,2,13, 16,18, 22-24 | C07D211/74<br>C08K5/3435 |
| X | PATENT ABSTRACTS OF JAPAN vol. 009, no. 012 (C-261)18 January 1985 & JP-A-59 161 448 ( ADEKA ARGUS KAGAKU ) 12 September 1984<br>*see formula I, X= option II, Y=option VI* | 1,7-9, 13,18, 22-24 | |
| A | EP-A-0 097 616 (CIBA-GEIGY) 4 January 1984 | 1-24 | |
| D,Y | EP-A-0 058 434 (ADEKA ARGUS CHEMICAL CO. LTD.) 25 August 1982<br>*see definition of Y and X* | 1-24 | |
| X<br>Y | EP-A-0 453 405 (CIBA-GEIGY AG) 23 October 1991 | 1,11,13, 18,22-24<br>1-24 | TECHNICAL FIELDS SEARCHED (Int. Cl.5 )<br>C07D<br>C08K |

The present search report has been drawn up for all claims

| Place of search | Date of completion of the search | Examiner |
|---|---|---|
| MUNICH | 29 NOVEMBER 1993 | SCRUTON-EVANS I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)